# EUROPÄISCHE PATENTSCHRIFT

(11) **EP 1 587 795 B1**
(45) Veröffentlichungstag und Bekanntmachung des Hinweises auf die Patenterteilung: **13.05.2009**
(21) Anmeldenummer: 04700987.3
(22) Anmeldetag: 09.01.2004
(51) Int. Cl.: C07D 239/80, C07D 243/02, C07D 471/04, C07D 285/36, C07D 249/08, C07D 403/12, C07D 417/12, C07D 405/12, A61K 31/505, A61K 31/55, A61K 31/435, A61K 31/54

(54) **N- (1-BENZYL-2-OXO-2- (1-PIPERAZINYL) ETHYL) -1-PIPERIDINCARBOXAMID-DERIVATE UND VERWANDTE VERBINDUNGEN ALS CGRP-ANTAGONISTEN ZUR BEHANDLUNG VON KOPFSCHMERZEN**
N- (1-BENZYL-2-OXO-2- (1-PIPERAZINYL) ETHYL) -1-PIPERIDINCARBOXAMID-DERIVATIVES AND RELATED COMPOUNDS USE AS CGRP-ANTAGONISTS FOR TREATING A HEADACHE
DERIVES DE N- (1-BENZYL-2-OXO-2- (1-PIPERAZINYL) ETHYLE) -1-PIPERIDINE-CARBOXAMIDE ET COMPOSES APPARENTES UTILISES COMME ANTAGONISTES DE CGRP ET DESTINES AU TRAITEMENT DES MAUX DE TETE

(30) Priorität: 14.01.2003 DE 10300973
(43) Veröffentlichungstag der Anmeldung: 26.10.2005
(73) Patentinhaber: Boehringer Ingelheim International GmbH, 55216 Ingelheim am Rhein (DE)
(72) Erfinder: BAUER, Eckhart, 88400 Biberach (DE); GERLACH, Kai, 89073 Ulm (DE); HURNAUS, Rudolf, 88400 Biberach (DE); MUELLER, Stephan Georg, 88447 Warthausen (DE); RUDOLF, Klaus, 88447 Warthausen (DE); SCHINDLER, Marcus, 88400 Biberach (DE); STENKAMP, Dirk, 88400 Biberach (DE)
(86) Internationale Anmeldenummer: PCT/EP2004/000087
(87) Internationale Veröffentlichungsnummer: WO 2004/063171

(56) Entgegenhaltungen:
- WO-A-01/10425
- WO-A-98/11128
- WO-A-03/104236
- WO-A-20/04000289
- MALLEE J J ET AL: "Receptor Activity-modifying Protein 1 Determines the Species Selectivity of Non-peptide CGRP Receptor Antagonists" JOURNAL OF BIOLOGICAL CHEMISTRY, AMERICAN SOCIETY OF BIOLOGICAL CHEMISTS, BALTIMORE, MD, US, Bd. 277, Nr. 16, 19. April 2002 (2002-04-19), Seiten 14294-14298, XP002271313 ISSN: 0021-9258

## Beschreibung

Gegenstand der vorliegenden Erfindung sind neue Carbonsäuren und deren Ester der allgemeinen Formel 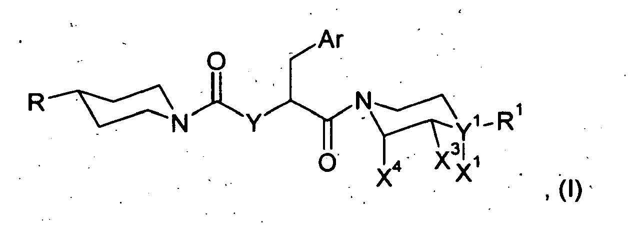 deren Tautomere, deren Diastereomere, deren Enantiomere, deren Gemische und deren Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

Die folgenden Anmeldungen beschreiben bereits Verbindungen mit CGRPantagonistischen Eigenschaften und reflektieren den allgemeinen Stand der Technik, jedoch unterscheiden sich die Verbindungen der vorliegenden Anmeldung strukturell von denen des Stands der Technik: WO 98/11128; WO 01/10425; WO 03/104236; WO 2004/000289; sowie Mallee et al., Journal of Biological Chemistry 2002 (16), Seiten 14294-14298.

In der obigen allgemeinen Formel I bedeuten
R die 2-Oxo-1,3,4,5-tetrahydro-1,3-benzodiazepin-3-yl-Gruppe,
Ar die 3,5-Dibrom-4-hydroxyphenyl-, 4-Amino-3,5-dibromphenyl-, 4-Brom-3,5-dimethylphenyl-, 3,5-Dichlor-4-methylphenyl-, 3,4-Dibromphenyl-, 3-Brom-4,5-dimethylphenyl-, 3,5-Dibrom-4-methylphenyl-, 3-Chlor-4-methylphenyl-, 3,4-Difluorphenyl-, 4-Hydroxyphenyl-, 1-Naphthyl-, 3,5-Dibrom-4-fluorphenyl-, 3,5-Bis-(trifluormethyl)-phenyl-, 3,4,5-Trimethylphenyl-, 3-(Trifluormethyl)-phenyl-, 3,5.Dimethyl-4-methoxyphenyl-, 4-Amino-3,5-dichlgrphenyl-, 2,4-Bis-(trifluormethyl)-phenyl-, 3,4,5-Tribromphenyl-, 3,4-Dimethoxyphenyl-, 3,4-Dichtorphenyl-, 4-Brom-3,5-dichlorphenyl-, 2-Naphthyl-, 2,3-Dihydrobenzo[b]fur-5-yl-, 1,2,3,4-Tetrahydro-1-naphthyl- oder 2,3-Dichlorphenylgruppe,
Y die Methylen- oder die -NH-Gruppe,
Y¹ das Kohlenstoff- oder das Stickstoffatom,
X¹ ein freies Elektronenpaar, wenn Y¹ das Stickstoffatom bedeutet, oder, sofern Y¹ das Kohlenstoffatom ist, das Wasserstoffatom, die Carbonsäure- oder die Methoxycarbonylgruppe und
R¹ einen Rest der allgemeinen Formel 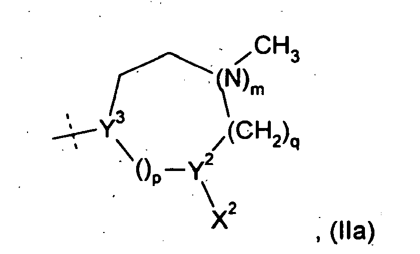 in der
Y² das Kohlenstoffatom oder, sofern m den Wert 0 annimmt, auch das Stickstoffatom,
Y³, das von Y¹ stets verschieden ist, das Kohlenstoff- oder das Stickstoffatom,
X² eine Gruppe der allgemeinen Formel

CH₂CO₂R² (III)

in der
R² das Wasserstoffatom oder einen geradkettigen oder verzweigten C₁₋₄-Alkylrest darstellt,
oder, sofern Y² das Kohlenstoffatom ist, auch das Wasserstoffatom oder die gegebenenfalls mit Methanol oder Ethanol veresterte Carbonsäuregruppe,
m die Zahlen 0 oder 1,
p und q jeweils die Zahlen 0, 1 oder 2,
wobei die Summe von m, p und q die Werte 1 oder 2 annehmen kann,
bedeuten,
oder einen der Reste 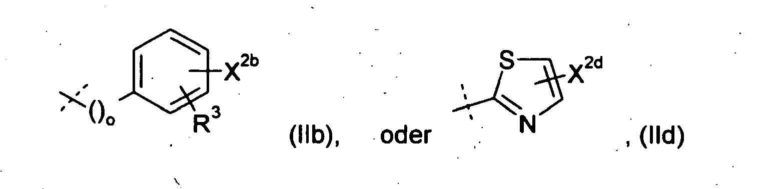 worin
X^{2b} das Wasserstoffatom oder die gegebenenfalls mit Methanol oder Ethanol veresterte Carbonsäuregruppe,
X^{2d} das Wasserstoffatom oder die gegebenenfalls mit Methanol veresterte Carbonsäuregruppe,
o die Zahlen 0, 1 oder 2 und
R³ das Wasserstoffatom oder die Trifluormethylgruppe darstellen,
wobei die vorstehend genannten Alkylgruppen oder die in den vorstehend genannten Resten enthaltenen Alkylgruppen, sofern nichts anderes angegeben wurde, 1 bis 4 Kohlenstoffatome enthalten und geradkettig oder verzweigt sein können,
deren Tautomere, deren Diastereomere, deren Enantiomere und deren Salze.

Als ganz besonders bevorzugte Verbindungen seien beispielsweise folgende genannt:
(1) 1'-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-[1,4']bipiperidinyl-4-essigsäuremethylester,
(2) 1'-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-[1,4']bipiperidinyl-4-essigsäure,
(3) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-piperidinessigsäureethylester,
(4) 4-{4-[4-Amino-3,5-dibrom-*N*-[[4-(2-oxo-1,3,4,5-tetrahydro-1,3-berizodiazepin-3-yl)-1-piperidinyl]carbonyl]-D-phenylalanyl]-1-piperazinyl}-benzoesäureethylester,
(5) 3-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-benzoesäureethylester,
(6) 4-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-benzoesäuremethylester,
(7) 4-{2-[1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl]-ethyl}benzoesäuremethylester,
(8) 4-{4-[4-Amino-3,5-dibrim-*N*-[[4-(2-oxo-1,3,4,5-tetrahydro-1,3-benzodiazepin-3-yl)-1-piperidinyl]carbonyl]-D-phenylalanyl]-1-piperazinyl}-3-(trifluormethyl)-benzoesäuremethylester,
(9) 3-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-benzoesäuremethylester,
(10) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-benzoesäure,
(11) 3-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-benzoesäure,
(12) 4-{-[4-Amino-3,5-dibrom-*N*-[[4-(2-oxo-1,3,4,5-terahydro-1,3-benzodiazepin-3-yl)--pipridinyl]carbonyl]-D-phenylalanyl]-4-piperidinyl}-benzoesäure,
(13) 4-{2-[1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl]-ethyl}benzoesäure,
(14) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-3-(trifluormethyl)-benzoesäure;
(15) 3-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-benzoesäure,
(16) 4-{1-[3-(1-Naphthyl)-*N*-[[4-(2-oxo-1,3,4,5-tetrahydro-1,3-benzodiazepin-3-yl)-1-piperidinyl]carbonyl]-D-alanyl]-4-piperidinyl}-1-piperazinessigsäureethylester,
(17) 4-{1-[3-(1-Naphthyl)-*N*-[[4-(2-oxo-1,3,4,5-tetrahydro-1,3-benzodiazepin-3-yl)-1-piperidinyl]carbonyl]-D-alanyl]-4-piperidinyl}-1-piperazinessigsäure,
(18) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-1-piperidinessigsäure,
(19) 4-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidipyl}-1-piperazinessigsäure-1,1-dimethylethylester,
(20) 4-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-1-piperazinessigsäureethylester,
(21) 4-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-1-piperazinessigsäure,
und deren Salze.

Die Verbindungen der allgemeinen Formel I werden nach prinzipiell bekannten Methoden hergestellt. Die folgenden Verfahren haben sich zur Herstellung der erfindungsgemäßen Verbindungen der allgemeinen Formel I besonders bewährt:

Zur Herstellung von Verbindungen der allgemeinen Formel (I), in der Y die NH-Gruppe bedeutet und weder X¹ noch X³ noch X⁴ noch R¹ eine freie Carbonsäurefunktion enthalten, im übrigen alle Reste wie eingangs erwähnt definiert sind:

Umsetzung von Piperidinen der allgemeinen Formel 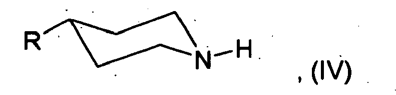 in der
R wie vorstehend definiert ist, mit Kohlensäurederivaten der allgemeinen Formel 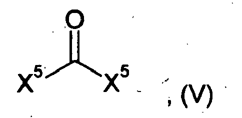 in der
X⁵ eine nukleofuge Gruppe, bevorzugt die 1*H*-Imidazol-1-yl-, 1*H*-1,2,4-Triazol-1-yl-, Trichlormethoxy- oder die 2,5-Dioxopyrrolidin-1-yloxygruppe, bedeutet,
und mit primären Aminen der allgemeinen Formel 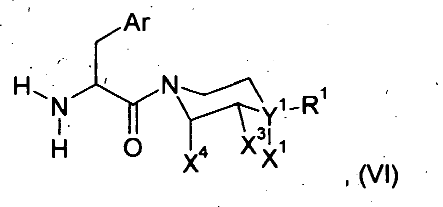 in der
weder X¹ noch X³ noch X⁴ noch R¹ eine freie Carbonsäurefunktion enthalten, im übrigen alle Reste wie oben definiert sind.

Die im Prinzip zweistufigen Reaktionen werden in der Regel als Eintopfverfahren durchgeführt, und zwar bevorzugt in der Weise, dass man in der ersten Stufe eine der beiden Komponenten (IV) oder (VI) mit äquimolaren Mengen des Kohlensäurederivats der allgemeinen Formel (V) in einem geeigneten Lösemittel bei tieferer Temperatur zur Reaktion bringt, anschließend wenigstens äquimolare Mengen der anderen Komponente (IV) oder (VI) zugibt und die Umsetzung bei höherer Temperatur beendet. Die Umsetzungen mit Bis-(trichlormethyl)-carbonat werden bevorzugt in Gegenwart von wenigstens 2 Äquivalenten (bezogen auf Bis-(trichlormethyl)-carbonat) einer tertiären Base, beispielsweise von Triethylamin, *N*-Ethyl-diisopropylamin, Pyridin, 1,5-Diazabicyclo[4,3,0]non-5-en, 1,4-Diazabicyclo-[2,2,2]octan oder 1,8-Diazabicyclo[5,4,0]undec-7-en, durchgeführt. Als Lösemittel, die wasserfrei sein sollten, kommen beispielsweise Tetrahydrofuran, Dioxan, Dimethylformamid, Dimethylacetamid, *N*-Methyl-2-pyrrolidon, 1,3-Dimethyl-2-imidazolidinon oder Acetonitril in Betracht, bei Verwendung von Bis-(trichlormethyl)-carbonat als Carbonylkomponente werden wasserfreie Chlorkohlenwasserstoffe, beispielsweise Dichlormethan, 1,2-Dichlorethan oder Trichlorethylen, bevorzugt. Die Reaktionstemperaturen liegen für die erste Reaktionsstufe zwischen -30 und +25°C, bevorzugt -5 und +10°C, für die zweite Reaktionsstufe zwischen +15°C und der Siedetemperatur des verwendeten Lösemittels, bevorzugt zwischen +20°C und +70°C (Siehe auch: H. A. Staab und W. Rohr, "Synthesen mit heterocyclischen Amiden (Azoliden)", Neuere Methoden der Präparativen Organischen Chemie, Band V, S. 53 - 93, Verlag Chemie, Weinheim/Bergstr., 1967; P. Majer und R.S. Randad, J. Org. Chem. 59, 1937 - 1938 (1994); K. Takeda, Y. Akagi, A. Saiki, T. Sukahara und H. Ogura, Tetrahedron Letters 24 (42), 4569 - 4572 (1983)).

b) Zur Herstellung von Verbindungen der allgemeinen Formel (I), in der Y die CH₂-Gruppe bedeutet und weder X¹ noch X³ noch X⁴ noch R¹ eine freie Carbonsäurefunktion enthalten, im übrigen alle Reste wie oben definiert sind:

Kupplung einer Carbonsäure der allgemeinen Formel 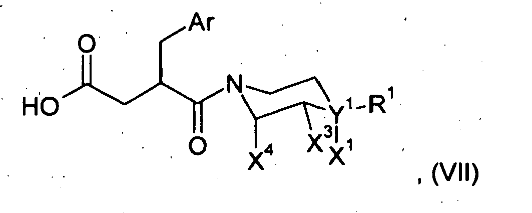 in der
weder X¹ noch X³ noch X⁴ noch R¹ eine freie Carbonsäurefunktion enthalten, im übrigen alle Reste wie oben definiert sind,
mit einem Piperidin der allgemeinen Formel 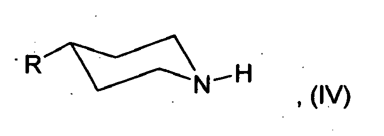 in der
R die eingangs erwähnten Bedeutungen besitzt.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropylcarbodiimid (DIC) oder Ethyl-(3-dimethylaminopropyl)-carbodiimid, *O*-(1*H*-Benzotriazol-1-yl)- *N,N-N',N'*-tetramethyluroniumhexafluorophosphat (HBTU) oder -tetrafluoroborat (TBTU) oder 1*H*-Benzotriazol-1-yl-oxy-tris-(dimethylamino)-phosphoniumhexafluorophosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenzes in Lösemitteln wie Dichlormethan, Tetrahydrofuran, Acetonitril, Dimethylformamid (DMF), Dimethylacetamid (DMA), *N*-Methylpyrrolidon (NMP) oder Gemischen aus diesen und bei Temperaturen zwischen -30 und +30°C, bevorzugt -20 und +25°C, durchgeführt. Sofern erforderlich, wird als zusätzliche Hilfsbase *N*-Ethyl-diisopropylamin (DIEA) (Hünig-Base) bevorzugt.

Als weiteres Kupplungsverfahren zur Synthese von Verbindungen der allgemeinen Formel (I) wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel (VII) und dem Kohlensäure-monoisobutylester erhalten wird. Die Herstellung dieses gemischten Anhydrids und die Kupplung mit Aminen erfolgt im Eintopfverfahren, unter Verwendung der vorstehend genannten Lösemittel und bei Temperaturen zwischen -20 und +25°C, bevorzugt 0 und +25°C.

c) Zur Herstellung von Verbindungen der allgemeinen Formel (I), in der Y die CH₂-Gruppe bedeutet und weder X¹ noch X³ noch X⁴ noch R¹ eine freie Carbonsäurefunktion enthalten, im übrigen alle Reste wie oben definiert sind:

Kupplung einer Verbindung der allgemeinen Formel 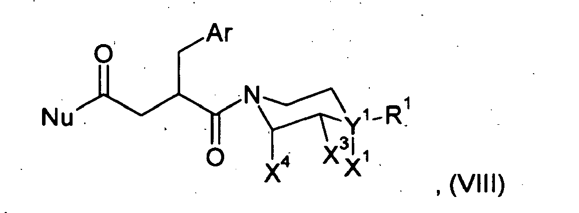 in der
weder X¹ noch X³ noch X⁴ noch R¹ eine freie Carbonsäurefunktion enthalten, im übrige alle Reste wie oben definiert sind, und Nu eine Austrittsgruppe, beispielsweise ein Halogenatom, wie das Chlor-, Brom- oder Iodatom, eine C₁₋₁₀-Alkylsulfonyloxygruppe, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch, 1 oder 2 Methylgruppen im Kohlenstoffgerüst substituierte 1*H*-Pyrazol-1-yl-, eine 1*H*-1,2,4-Triazol-1-yl-, 1*H*-1,2,3-Triazol-1-yl-, 1*H*-1,2,3,4-Tetrazol-1-yl-, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1*H*)-Oxopyridin-1-yl-oxy-, 2,5-Dioxopyrrolidin-1-yloxy-, Phthalimidyloxy-, 1*H*-Benzo-triazol-1-yloxy- oder Azidgruppe bedeutet,
mit einem Piperidin der allgemeinen Formel 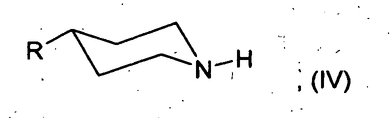 in der
R wie eingangs erwähnt definiert ist.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, N-Ethyl-diisopropylamin, *N*-Ethyl-dicyclohexylamin, 1,4-Diazabicyclo[2,2,2]octan oder 1,8-Diazabicyclo-[5,4,0]undec-7-en, als Lösemittel beispielsweise Dichlormethan, Tetrahydrofuran, 1,4-Dioxan, Acetonitril, Dimethylformamid, Dimethylacetamid, *N*-Methylpyrrolidon oder Gemische davon in Betracht;, werden als Hilfsbasen Alkali- oder Erdalkalihydroxide, Alkalicarbonate oder -acetate verwendet, kann dem Reaktionsgemisch auch Wasser als Cosolvens zugesetzt werden.

d) Zur Herstellung von Verbindungen der allgemeinen Formel (I), in der weder X¹ noch X³ noch X⁴ noch R¹ eine freie Carbonsäurefunktion enthalten, im übrigen alle Reste wie oben definiert sind:

Kupplung einer Carbonsäure der allgemeinen Formel 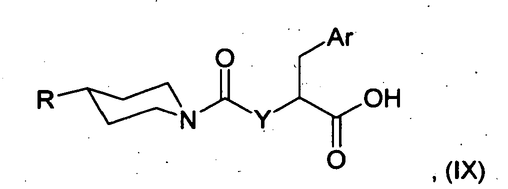 in der

Ar, R und Y wie oben definiert sind,
mit einem cyclischen, sekundären Amin der allgemeinen Formel 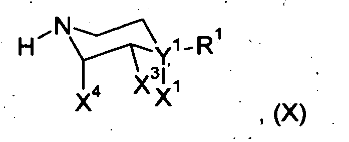 in der
weder X¹ noch X³ noch X⁴ noch R¹ eine freie Carbonsäurefunktion enthalten, die Reste im übrigen wie eingangs definiert sind.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropylcarbodiimid (DIC) oder Ethyl-(3-dimethylaminopropyl)-carbodiimid, *O*-(1*H*-Benzotriazol-1-yl)- *N,N-N',N*'-tetramethyluroniumhexafluorophosphat (HBTU) oder -tetrafluoroborat (TBTU) oder 1*H*-Benzotriazol-1-yl-oxy-tris-(dimethylamino)-phosphoniumhexafluorophosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenzes in Lösemitteln wie Dichlormethan, Tetrahydrofuran, Acetonitril, Dimethylformamid (DMF), Dimethylacetamid (DMA), *N*-Methylpyrrolidon (NMP) oder Gemischen aus diesen und bei Temperaturen zwischen -30 und +30°C, bevorzugt -20 und +25°C, durchgeführt. Sofern erforderlich, wird als zusätzliche Hilfsbase *N*-Ethyl-diisopropylamin (DIEA) (Hünig-Base) bevorzugt.

Als weiteres Kupplungsverfahren zur Synthese von Verbindungen der allgemeinen Formel (I) wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel (IX) und dem Kohlensäure-monoisobutylester erhalten wird. Die Herstellung dieses gemischten Anhydrids und die Kupplung mit den Aminen der allgemeinen Formel (X) erfolgt im Eintopfverfahren, unter Verwendung der vorstehend genannten Lösemittel und bei Temperaturen zwischen -20 und +25°C, - bevorzugt 0 und +25°C.

e) Zur Herstellung von Verbindungen der allgemeinen Formel (I), in der weder X¹ noch X³ noch X⁴ noch R¹ eine freie Carbonsäurefunktion enthalten, im übrigen alle Reste wie oben definiert sind:

Kupplung einer Verbindung der allgemeinen Formel 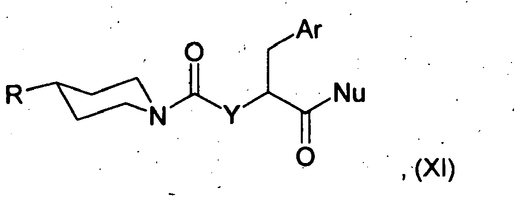 in der
Ar, R und Y wie oben definiert sind und Nu eine Austrittsgruppe, beispielsweise ein Halogenatom, wie das Chlor-, Brom- oder Iodatom, eine C₁₋₁₀-Alkylsulfonyloxygruppe, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch 1 oder 2 Methylgruppen im Kohlenstoffgerüst substituierte 1*H*-Pyrazol-1-yl-, eine 1*H*-1,2,4-Triazol-1-yl-, 1*H*-1,2,3-Triazol-1-yl-, 1*H*-1,2,3,4-Tetrazol-1-yl-, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1*H*)-Oxopyridin-1-yl-oxy-, 2,5-Dioxopyrrolidin-1-yloxy-, Phthalimidyloxy-, 1*H*-Benzotriazol-yloxy- oder Azidgruppe bedeutet,
mit einem cyclischen, sekundären Amin der allgemeinen Formel 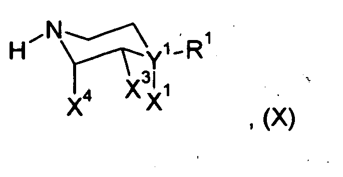 in der
weder X¹ noch X³ noch X⁴ noch R¹ eine freie Carbonsäurefunktion enthalten, die Reste im übrigen wie eingangs definiert sind.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt. Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, *N*-Ethyl-diisopropylamin, *N*-Ethyl-dicyclohexylamin, 1,4-Di-azabicyclo[2,2,2]octan oder 1,8-Diazabicyclo[5,4,0]undec-7-en, als Lösemittel beispielsweise Dichlormethan, Tetrahydrofuran, 1,4-Dioxan, Acetonitril, Dimethylformamid, Dimethylacetamid, *N*-Methylpyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.

f) Zur Herstellung von Verbindungen der allgemeinen Formel (I), in der X¹, X³, X⁴ oder R¹ eine freie Carbonsäurefunktion enthalten, im übrigen alle Reste wie eingangs definiert sind:

Hydrolyse von unter die allgemeine Formel (I) fallenden Carbonsäureestern, bei denen entweder X¹ oder X³ oder X⁴ oder R¹ eine Carbonsäureesterfunktion enthalten und alle übrigen Reste wie eingangs definiert sind. Die Hydrolyse kann unter saurer oder alkalischer Katalyse unter dem Fachmann geläufigen Bedingungen durchgeführt werden. Die sauer katalysierte Hydrolyse erfolgt in Gegenwart starker organischer oder anorganischer Säuren, beispielsweise Methansulfonsäure, *p*-Toluolsulfonsäure, Salzsäure, Bromwasserstoffsäure oder Schwefelsäure, bevorzugt in Gegenwart von mit Wasser mischbaren Solventien, beispielsweise Methanol, Ethanol oder 1,4-Dioxan, und bei Temperaturen zwischen 0°C und der Siedetemperatur des Hydrolysegemisches. Vorteilhaft ist es, die unter die allgemeine Formel (I) fallenden Carbonsäureester, gegebenenfalls auch in Gegenwart mit Wasser mischbarer Cosolvenzien, alkalisch zu verseifen. Zur Durchführung wird, bezogen auf den jeweiligen Carbonsäureester, wenigstens 1 Äquivalent einer anorganischen Base, beispielsweise wässerige Lithiumhydroxid-Lösung, Natron-, Kali- oder Barytlauge, eingesetzt. Geeignete Temperaturen liegen zwischen 0°C und 50°C, wobei Zimmertemperatur bevorzugt wird. Aus dem zunächst erhaltenen Salz lässt sich durch Ansäuern in bekannter Weise die gesuchte Säure freisetzen.

Die erfindungsgemäßen neuen Carbonsäuren und Carbonsäureester der allgemeinen Formel (I) enthalten ein oder mehrere Chiralitätszentren. Sind beispielsweise zwei Chiralitätszentren vorhanden, dann können die Verbindungen in Form zweier diastereomerer Antipodenpaare auftreten. Die Erfindung umfasst die einzelnen Isomeren ebenso wie ihre Gemische.

Die Trennung der jeweiligen Diastereomeren gelingt auf Grund ihrer unterschiedlichen, physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)-Camphersulfonsäure, bzw. einer optisch aktiven Base, beispielsweise mit (R)-(+)-1-Phenylethylamin, (S)-(-)-1-Phenylethylamin oder (S)-Brucin, entstehen.

Nach einem üblichen Verfahren zur Isometentrennung wird das Racemat einer Verbindung der allgemeinen Formel (I) mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichende unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel I fallender diastereomerer Verbindungen wird auch dadurch erhalten, dass man die oben beschriebenen Synthesen mit jeweils einer geeigneten (R)- bzw. (S)-konfgurierten Reaktionskomponente durchführt.

Die Ausgangsverbindungen der allgemeinen Formel (IV) erhält man, soweit sie nicht literaturbekannt oder gar käuflich sind, entsprechend den in WO 98/11128 und DE 199 52 146 angegebenen Verfahren. Die Ausgangsverbindungen der allgemeinen Formel (V) sind käuflich. Verbindungen der allgemeinen Formel (VI) lassen sich nach dem Peptidchemiker geläufigen Methoden aus geschützten Phenylalaninen und Aminen der allgemeinen Formel (X) herstellen. Die Ausgangsverbindungen der allgemeinen Formel (VII) erhält man beispielsweise durch Umsetzung von cyclischen, sekundären Aminen der allgemeinen Formel (X) mit 2-(Alkoxycarbonylmethyl)-3-aryl-propansäuren und anschließende hydrolytische Abspaltung der Alkylgruppe. Die erforderlichen 2-(Alkoxycarbonylmethyl)-3-arylpropansäuren können in Analogie zu literaturbekannten Methoden (Saul G. Cohen und Aleksander Milovanovic, J. Am. Chem. Soc. 90, 3495-3502 [1968]; Hiroyuki Kawano, Youichi Ishii, Takao Ikariya, Masahiko Saburi, Sadao Yoshikawa, Yasuzo Uchida, und Hidenori Kumobayashi, Tetrahedron Letters 28, 1905-8 [1987]) hergestellt werden. Carbonsäuren der allgemeinen Formel IX sind in WO 98/11128 beschrieben worden oder können nach den dort angegebenen Verfahren aus allgemeine zugänglichen Ausgangsmaterialien hergestellt werden Die cyclischen, sekundären Amine der allgemeinen Formel (X) lassen sich aus Verbindungen der allgemeinen Formel 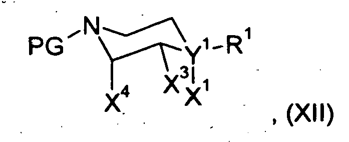 worin PG eine abspaltbare Schutzgruppe darstellt, synthetisieren, beispielsweise durch Hydrogenolyse einer Phehylmethylgruppe. Die Vorprodukte zur Synthese der Verbindungen der allgemeinen Formel (XII) sind aus käuflichen oder nach gängigen Verfahren leicht erhältlichen Ausgangsmaterialien zugänglich. Die Ausgangesverbindungen der allgemeinen Formeln VIII und XI schließlich können nach bekannten Vorbildern aus den entsprechenden Carbonsäuren (VII) bzw. (IX) bereitet werden.

Die erhaltenen Verbindungen der allgemeinen Formel (I) können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich' die neuen Verbindungen der Formel (I), falls sie Carbonsäurefunktion enthalten, gewünschtenfalls in ihre Additionssalze mit anorganischen oder organischen Basen, insbesondere für die pharmazeutische Anwendung in ihre physiologisch verträglichen Additionssalze überführen. Als Basen kommen hierfür beispielsweise Natriumhydroxid, Kaliumhydroxid, Ammoniak, Cyclohexylamin, Dicyclohexylamin, Ethanolamin, Diethanolamin und Triethanolamin in Betracht.

Die neuen Verbindungen der allgemeinen Formel (I) und deren physiologisch verträglichen Salze besitzen CGRP-antagonistische Eigenschaften, und zeigen gute Affinitäten in CGRP-Rezeptorbindungsstudien. Die Verbindungen weisen in den nachstehend beschriebenen pharmakologischen Testsystemen CGRP-antaganistische Eigenschaften auf.

Zum Nachweis der Affinität von Verbindungen der allgemeinen Formel I zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, NaHCO₃ 16.2, MgSO₄ 0.8, NaHPO₄ 1.0, CaCl₂ 1.8, D-Glucose 55, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und 10 Minuten lang bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40, angereichert mit 1% Rinderserum-Albumin und 0.1% Bacitracin) rezentrifugiert und resuspendiert (1 ml / 1000000 Zellen). Das Homogenat wird bei -80°C eingefroren. Die Membranpräparationen sind bei diesen Bedingungen für mehr als 6 Wochen ,stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden 180 Minuten lang bei Raumtemperatur mit 50 pM ¹²⁵I-lodotyrosyl-Calcitonine-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität in Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die Verbindungen der allgemeinen Formel (I) zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C 15 Minuten lang vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C, 15 Minuten lang) extrahiert. Die Überstände werden in flüssigem Stickstoff ein-gefroren und bei -20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die Verbindungen der allgemeinen Formel (I) zeigen in dem beschriebenen in-vitro-Testmodell CGRP-antagonistische Eigenschaften in einem Dosisbereich zwischen 10⁻¹¹ bis 10⁻⁵ M.

Auf Grund ihrer pharmakologischen Eigenschaften eignen sich die Verbindungen der allgemeinen Formel I und deren Salze mit physiologisch verträglichen Säuren bzw. Basen somit zur akuten und prophylaktischen Behandlung von Kopfschmerzen, insbesondere Migräne- bzw. Cluster-Kopfschmerz. Weiterhin beeinflussen die Verbindungen der allgemeinen Formel (I) auch die folgenden Erkrankungen positiv: "complex regional pain syndrome", nicht-insulinabhängigen Diabetes mellitus ("NIDDM"), cardiovaskuläre Erkrankungen, Morphintoleranz, Clostritiumtoxin-bedingte Durchfallerkrankungen, Erkrankungen der Haut, insbesondere thermische und strahlenbedingte Hautschäden inklusive Sonnenbrand, entzündliche Erkrankungen, z.B. entzündliche Gelenkerkrankungen (Arthritis), entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis. Die Symptomatik menopausaler, durch Gefäßerweiterung und erhöhten Blutfluss verursachter Hitzewallungen östrogendefizienter Frauen wird durch die CGRP-Antagonisten der vorliegenden Anwendung präventiv und akut-therapeutisch günstig beeinflusst,
wobei sich dieser Therapieansatz vor der Hormonsubstitution durch Nebenwirkungsarmut auszeichnet. Darüber hinaus zeigen die Verbindungen der allgemeinen Formel I eine lindernde Wirkung auf Schmerzzustände im allgemeinen.

Die zur Erzielung einer entsprechenden Wirkung erforderliche Dosierung beträgt zweckmäßigerweise bei intravenöser oder subkutaner Gabe 0.001 bis 30 mg/kg Körpergewicht, vorzugsweise 0.01 bis 5 mg/kg Körpergewicht, und bei oraler, nasaler oder inhalativer Gabe 0.01 bis 50 mg/kg Körpergewicht, vorzugsweise 0.1 bis 30 mg/kg Körpergewicht, jeweils 1 bis 3 x täglich.

Hierzu lassen sich die erfindungsgemäß hergestellten Verbindungen der allgemeinen Formel I, gegebenenfalls in Kombination mit anderen Wirksubstanzen, wie z.B. Antiemetica, Prokinetica, Neuroleptica, Antidepressiva, Neurokinin-Antagonisten, Anticonvulsiva, Histamin-H1-Rezeptorantagonisten, Antimuscarinica, β-Blockern, α-Agonisten und α-Antagonisten, Ergotalkaloiden, schwachen Analgetica, nichtsteroidalen Antiphlogistica, Corticosteroiden, Calcium-Antagonisten, 5-HT_{1D}-Agonisten oder anderen Antimigränemitteln, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen einarbeiten.

Für die oben erwähnten Kombinationen kommen somit als weitere Wirksubstanzen beispielsweise Meloxicam, Ergotamin, Dihydroergotamin, Metoclopramid, Domperidon, Diphenhydramin, Cyclizin, Promethazin, Chlorpromazin, Dexämethason, Flunarizin, Dextropropoxyphen, Meperidin, Propranolol, Nadolol, Atenolol, Clonidin, Indoramin, Carbamazepin, Phenytoin, Valproat, Amitryptilin, Lidocain, Diltiazem oder Sumatriptan und andere 5-HT_{1D}-Agonisten wie z.B. Naratriptan, Zolmitriptan, Avitriptan, Rizatriptan und Eletriptan in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der Verbindungen der allgemeinen Formel (I) als wertvolle Hilfsmittel zur Erzeugung und Reinigung (Affinitätschromatographie) von Antikörpern sowie, nach geeigneter radioaktiver Markierung, beispielsweise durch direkte Markierung mit ¹²⁵I oder ¹³¹I oder durch Tritiierung geeigneter Vorstufen, beispielsweise durch Ersatz von Halogenatomen durch Tritium, in RIA- und ELISA-Assays und als diagnostische bzw. analytische Hilfsmittel in der Neurotransmitter-Forschung.

### Die nachfolgenden Beispiele sollen die Erfindung näher erläutern:

### Vorbemerkungen:

Die Verbindungen wurden teils nach klassischen Syntheseverfahren, teils unter Verwendung von Methoden der Kombinatorischen Chemie hergestellt.

Als Syntheseautomat diente das Gerät ASW2000 der Firma Chemspeed Ltd., Rheinstraße 32, CH-4302 Augst, Schweiz.

Für alle nach klassischen Methoden hergestellten Verbindungen IR-, ¹H-NMR und in der Regel auch Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Falls nähere Angaben zur Konfiguration fehlen, bleibt offen, ob es sich um reine Enantiomere handelt oder ob partielle oder gar völlige Racemisierung eingetreten ist. Zur Chromatographie wurden die folgenden Fließmittel bzw. Fließmittelgemische verwendet:
- FM A =: Essigsäureethylester/Methanol 100/5 v/v
- FM B =: Essigsäureethylester/Methanol 9/1 v/v
- FM C =: Essigsäureethylester/Methanol/konz. Ammoniak 80/20/1 v/v/v
- FM D =: Dichlormethan/Cyclohexan/Methanol/konz. Ammoniak 70/15/15/2 v/v/v/v
- FM E =: Essigsäureethylester/Eisessig 99/1 v/v
- FM F =: Essigsäureethylester/Methanol/Eisessig 90/10/1 v/v/v
- FM G =: Dichlormethan/Methanol/konz. Ammoniak 90/9/1 v/v/v
- FM H =: Petrolether/Essigsäureethylester 4/6 v/v
- FM I =: Dichlormethan/Methanol/Eisessig 90/10/2.5 v/v/v
- FM K =: Dichlormethan/Isopropanol 9/1 v/v
- FM M =: Dichlormethan/Methanol/konz. Ammoniak 75/25/5 v/v/v
- FM N =: Dichlormethan/Essigsäureethylester 1/1 v/v
- FM O =: Dichlormethan/Methanol 9/1 v/v
- FM P =: Dichlormethan/Essigsäureethylester/Cyclohexan/Methanol/konz. Ammoniak 60/16/5/5/0.6 v/v/v/v/v
- FM Q =: Dichlormethan/Methanol/konz. Ammoniak 80/20/2 v/v/v
- FM R =: Dichlormethan/Methanol/Eisessig 80/20/1 v/v/v
- FM S =: Dichlormethan/Methanol 9/1 v/v (Alox-DC-Platten [E. Merck, Darmstadt])
- FM T =: Dichlormethan/Methanol/Eisessig 70/30/3 v/v/v
- FM U =: Essigsäureethylester/Petrolether 2/1 v/v
- FM V =: Essigsäureethylester/Petrolether 1/4 v/v
- FM W =: Essigsäureethylester/Petrolether 3/7 v/v
- FM X =: Petrolether/Essigsäureethylester/Eisessig 8/2/0.5 v/v/v
- FM Y =: Essigsäureethylester/Petrolether 1/9 v/v
- FM Z =: Toluol/Petrolether/Essigsäureethylester 5/5/2 v/v/v
- FM AA =: Essigsäureethylester/Petrolether/Triethylamin 5/5/0.1 v/v/v
- FM BB =: Dichlormethan/Methanol 3/1 v/v (Alox-DC-Platten [E. Merck, Darmstadt])
- FM DD =: Essigsäureethylester/Methanol/konz. Ammoniak 70/30/3 v/v/v
- FM EE =: Dichlormethan/Ethanol 9/1 v/v
- FM FF =: Dichlormethan/Ethanol 50/1 v/v
- FM GG: = Dichlormethan/Ethanol 40/1 v/v
- FM HH =: Dichlormethan/Methanol 5/1 v/v
- FM II =: Essigsäureethylester/Methanol/konz. Ammoniak 90/10/1 v/v/v
- FM KK =: Essigsäureethylester/Methanol/konz. Ammoniak 60/40/4 v/v/v
- FM LL =: Essigsäureethylester/Methanol/konz. Ammoniak 50/50/5 v/v/v
- FM MM: = Essigsäureethylester/Cyclohexan 1/1 v/v
- FM NN =: Essigsäureethylester/Cyclohexan 2/8 v/v
- FM OO =: Dichlormethan/Methanol/konz. Ammoniak 70/30/3 v/v/v

In der Versuchsbeschreibung werden die folgenden Abkürzungen verwendet:
- Fp.:: Schmelzpunkt
- (Z):: (Zersetzung)
- DIEA:: *N*,*N*-Diisopropylethylamin
- Boc:: (1,1-Dimethylethoxy)carbonyl
- TBTU:: 2-(1*H*-Benzotriazol-1-yl)-1,1,3,3-tetramethyluronium-tetrafluoroborat
- HOBt:: 1-Hydroxybenzotriazol-hydrat
- CDT:: 1,1'-Carbonyldi-(1,2,4-triazol)
- PyBroP:: Bromo-tris-pyrrolidino-phosphoniumhexafluorophosphat
- HATU:: *O*-(7-Azabenzotriazol-1-yl)-*N*,*N*,*N*',*N*'-tetramethyluroniumhexafluorophosphat
- THF:: Tetrahydrofuran
- DMF:: Dimethylformamid
- EE:: Essigsäureethylester
- PE:: Petrolether
- LM:: Lösemittel
- ZT: Zimmertemperatur
- Lfd. Nr.:: Laufende Nummer

Die Bedeutung der in den Beispielen verwendeten, aus Buchstaben und Zahlen zusammengesetzten Symbole ergibt sich aus der folgenden Übersicht:

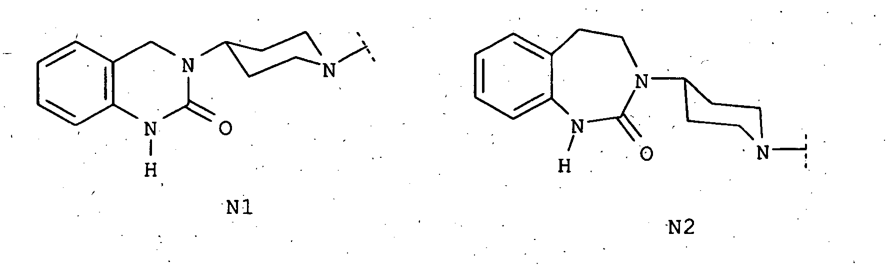

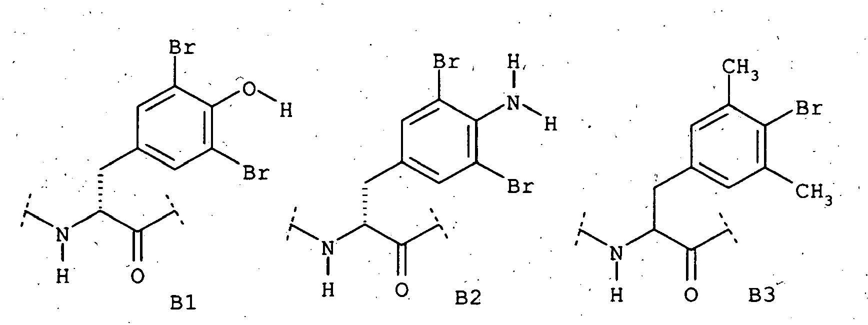

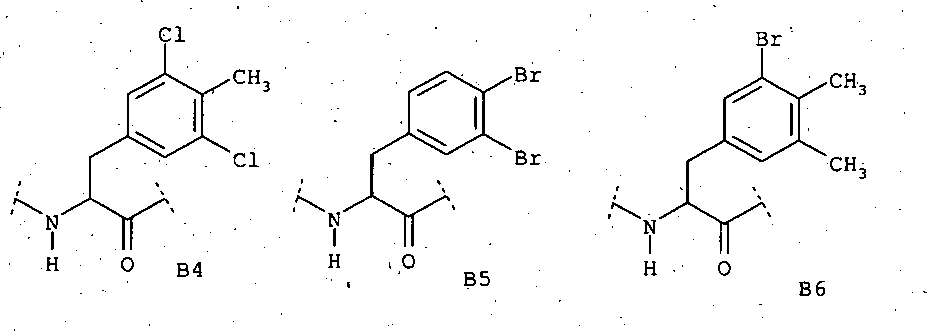

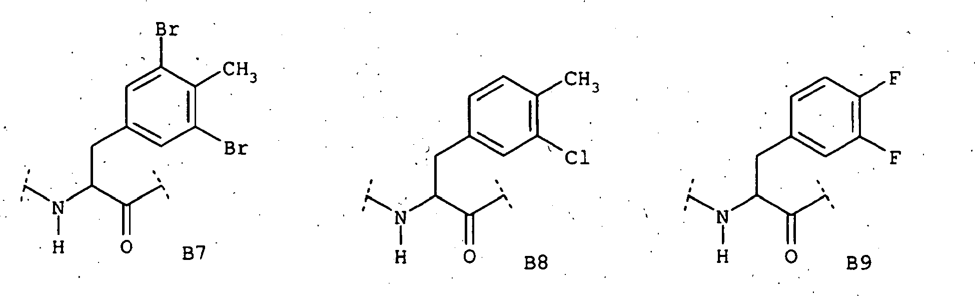

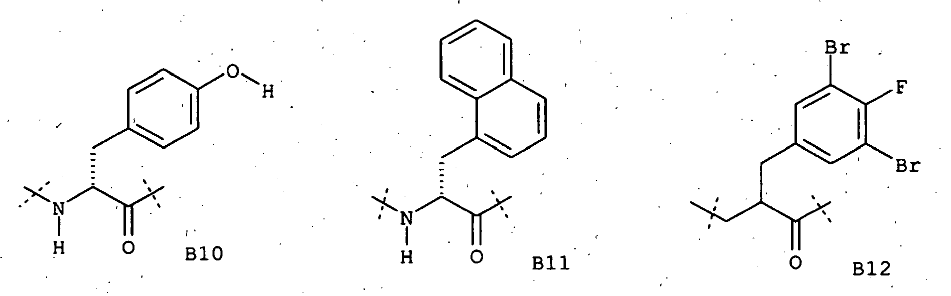

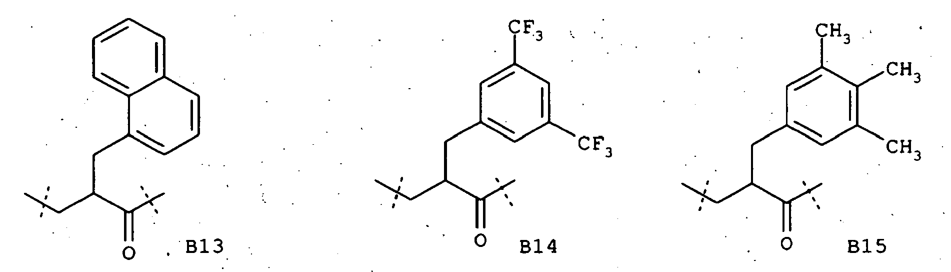

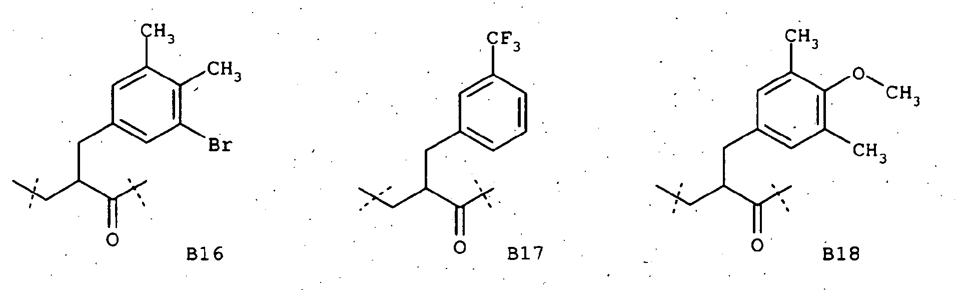

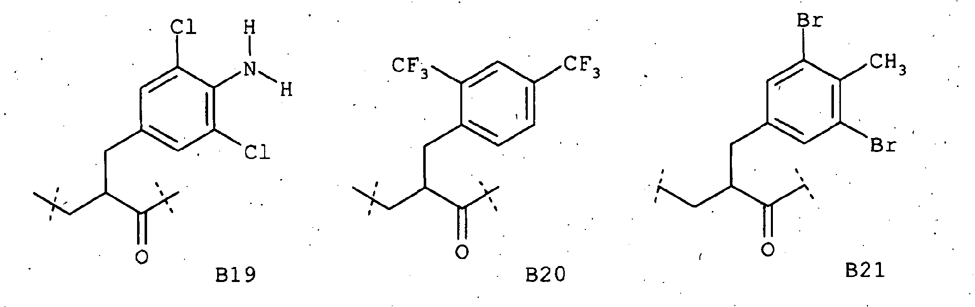

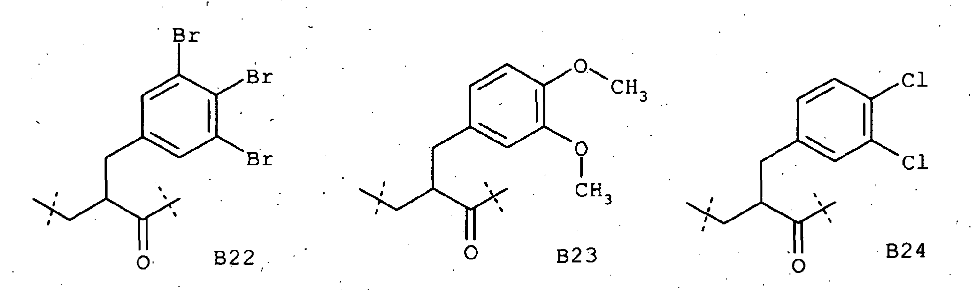

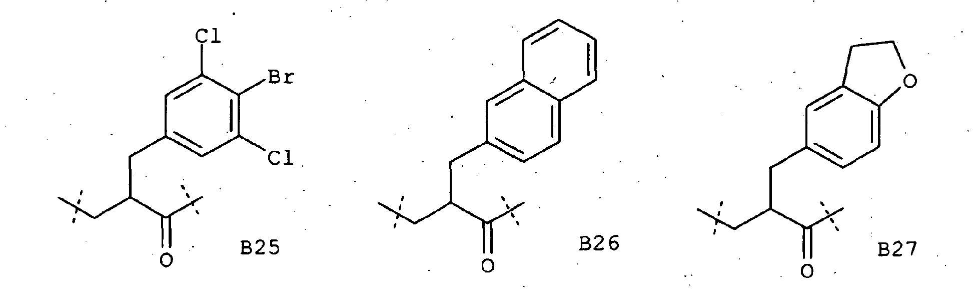

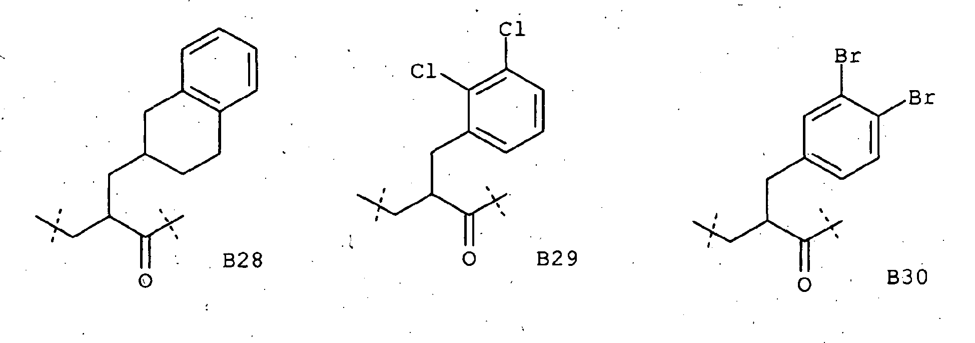

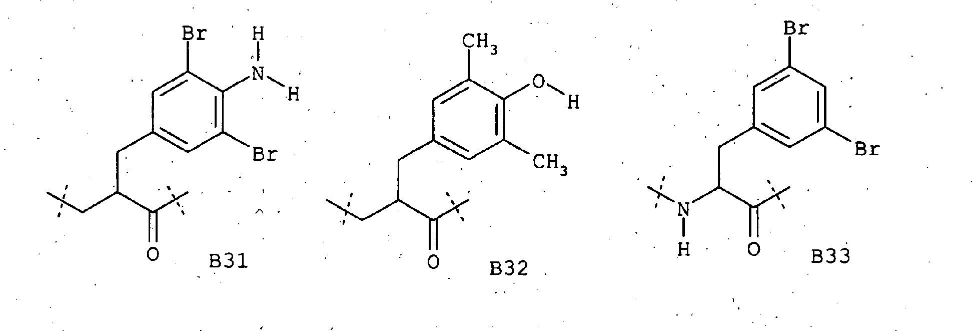

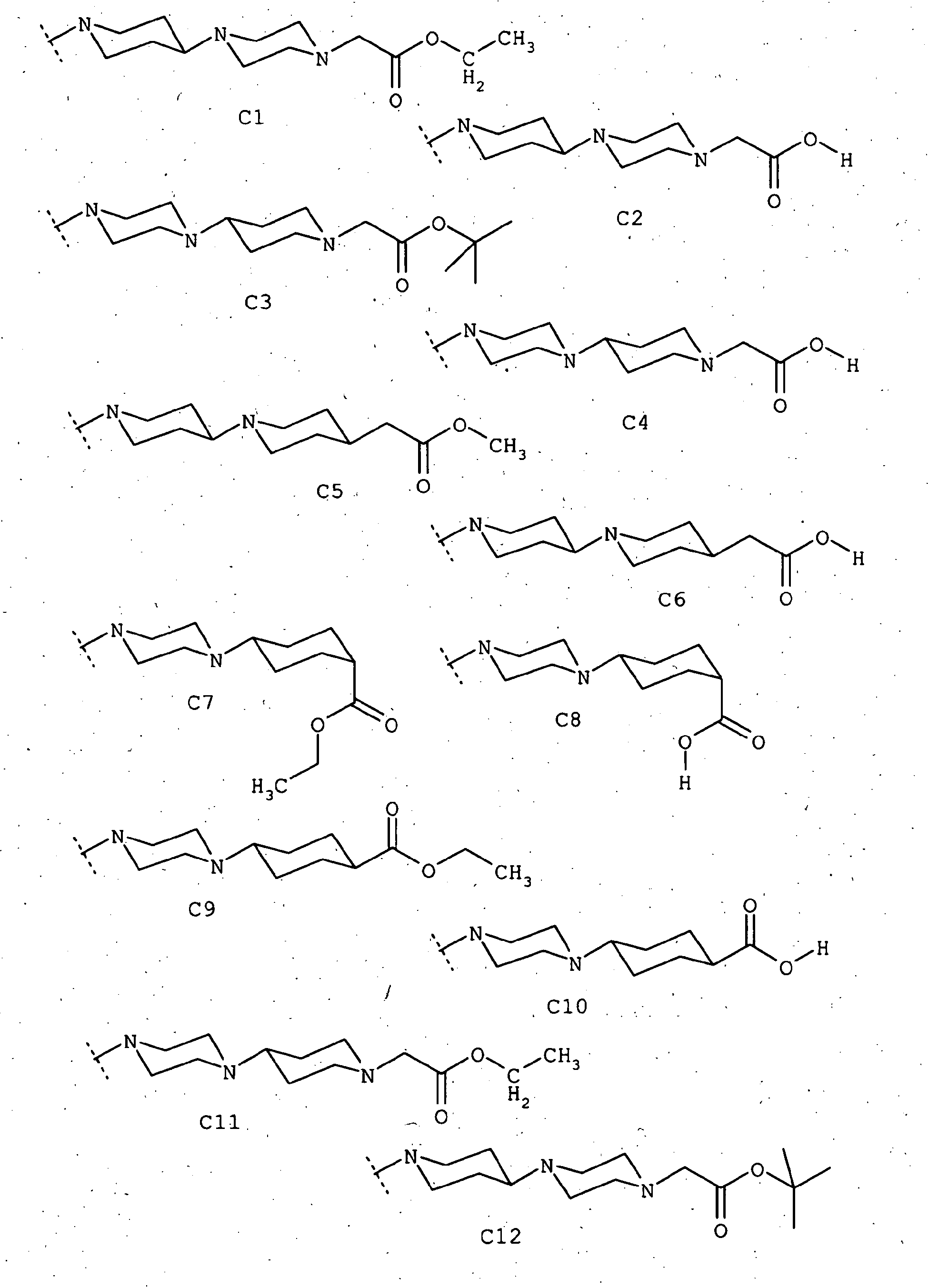

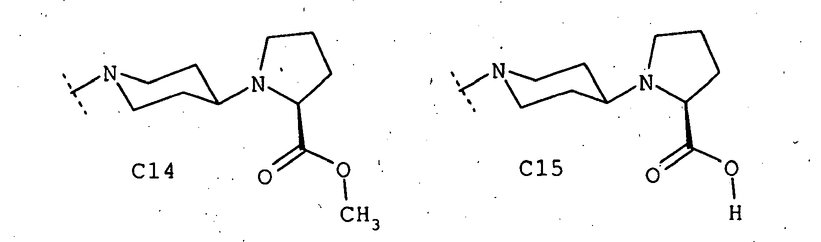

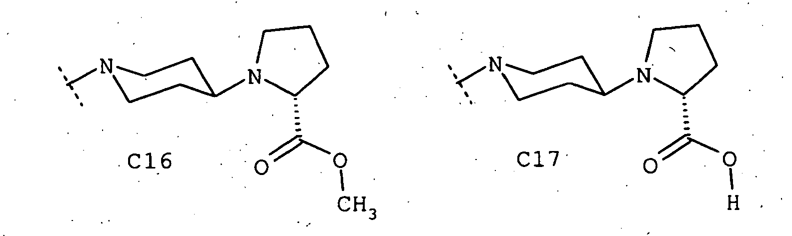

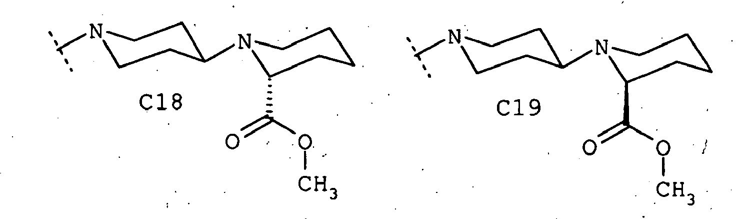

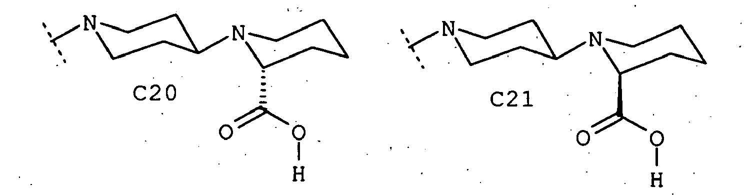

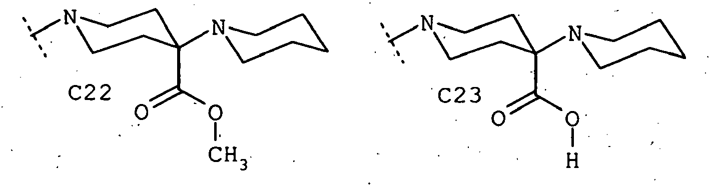

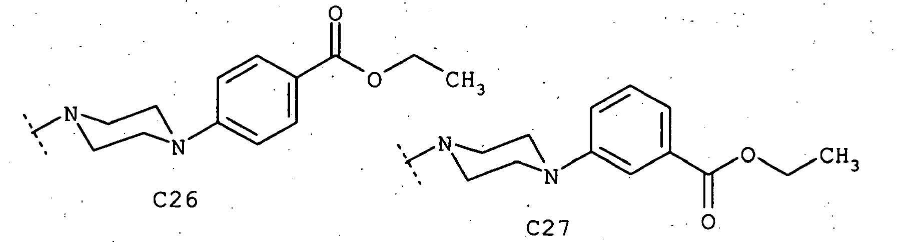

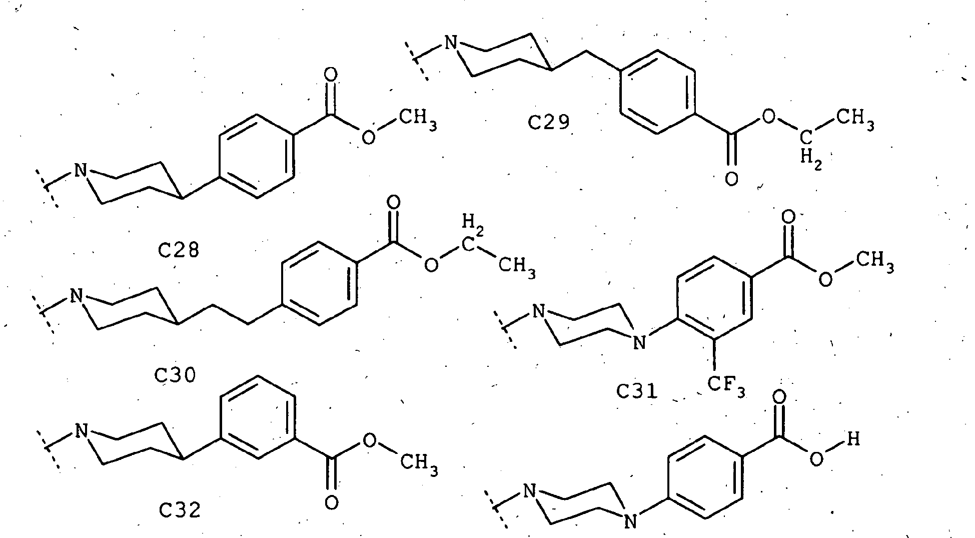

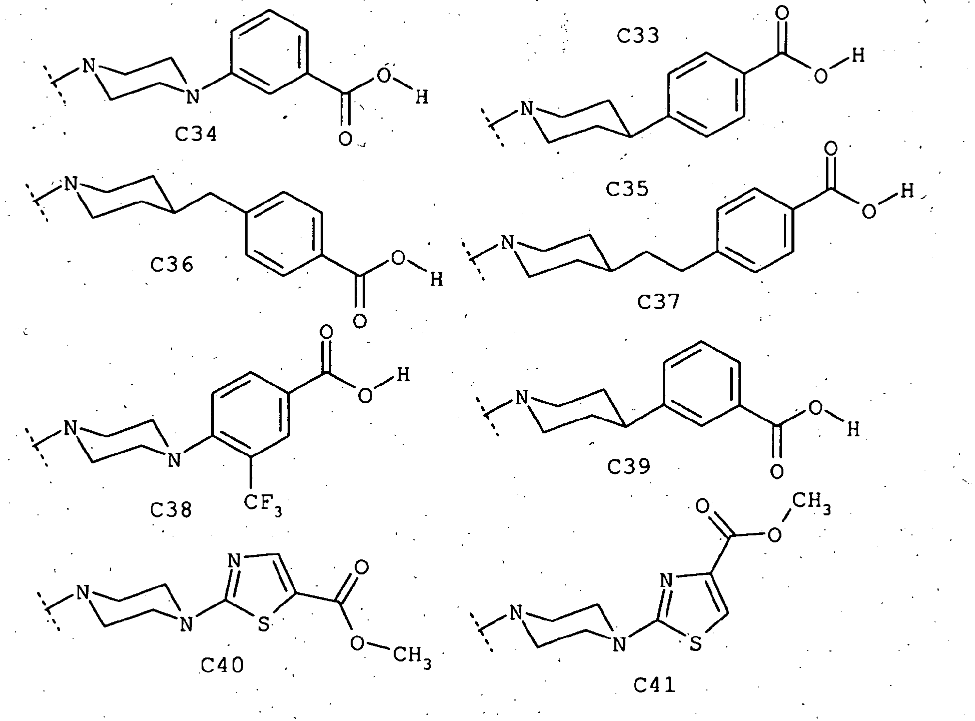

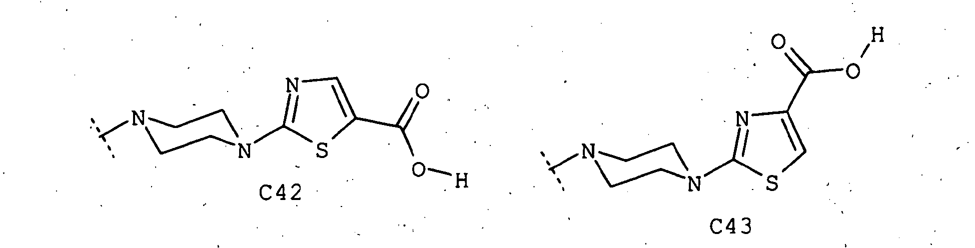

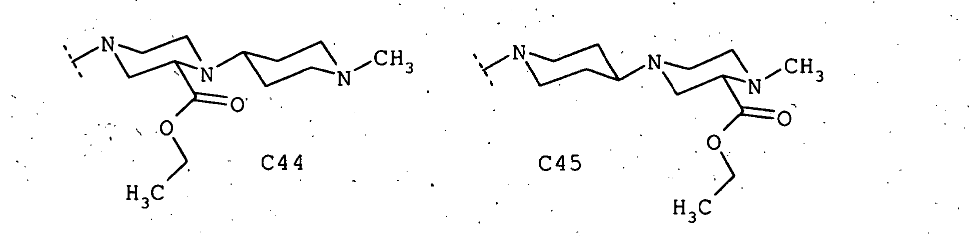

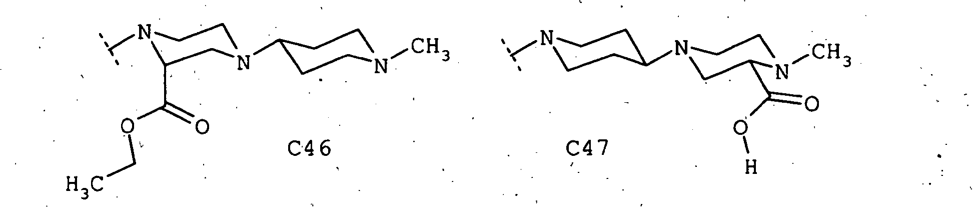

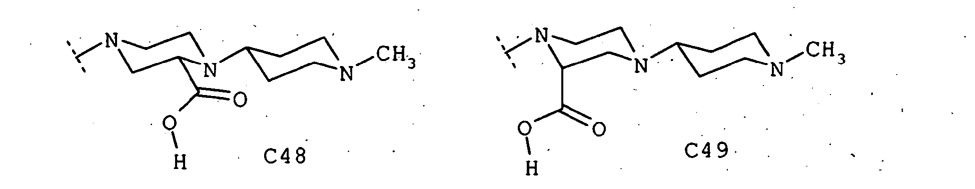

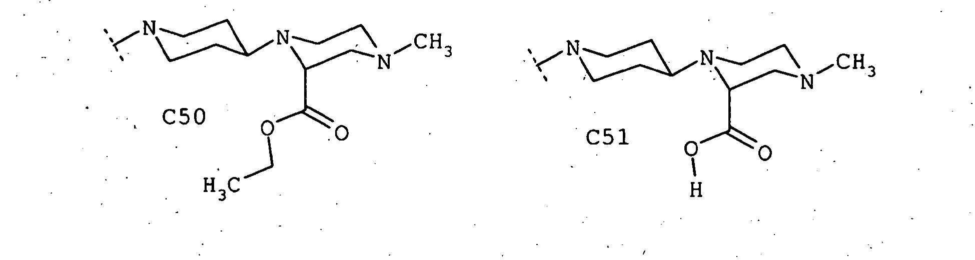

### A. Herstellung von Zwischenverbindungen

### (Referenz)Beispiel A1

### (R,S)-3,4-Dibrom-N-[[4-(3,4-dihydro-2(1H)-oxochinazolin-3-yl)-1-piperidinyl]carbonyl]-phenylalanin

Zu der Lösung von 20.0 g (0.033 mol) (*R*,*S*)-3,4-Dibrom-*N*-[[4-(3,4-dihydro-2(1*H*)-oxochinazolin-3-yl)-1-piperidinyl]carbonyl]-phenylalaninethylester in 500 ml Ethanol wurden 150 ml 1M Natronlauge gegeben und die Mischung anschließend 3.5 Stunden bei Zimmertemperatur gerührt. Das Lösemittel wurde am Rotationsverdampfer entfernt und der Rückstand mit 1 M Salzsäure sauer gestellt. Der ausgefallene Niederschlag wurde abgenutscht, gründlich mit Wasser gewaschen und im Umlufttrockenschrank bei 70°C getrocknet. Man erhielt 10.0 g (52% der Theorie) der gesuchten farblosen, kristallinen Substanz vom R_{f} 0.62 (FM M). IR (KBr): 1705, 1645 cm⁻¹ (C=O)

Entsprechend wurden die folgenden Verbindungen der allgemeinen Formel N-B-C hergestellt:

| **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|
| N2 | B2 | OH | aus N2-CO-B2-OMe mit 1 M LiOH, dann aq. 1M HCl | 96 | M | 0.49 | ESI: (M-H)⁻ = 606/608/610 (Br₂) | 1724, 1660 (C=O) | farblose Kristalle |

### (Referenz)Beispiel A2

### 3,4-Dibrom-N-[[4-(3,4-dihydro-2(1H)-oxochinazolin-3-yl)-1-piperidinyl]carbonyl]-D,L-phenylalaninethylester

Zu einer eisgekühlten Suspension von 18.0 g (0.051 Mol) (*R*,*S*)- 3,4-Dibrom-phenylalaninethylester in 300 ml THF gab man 9.7 g (0.056 Mol) CDT. Die Reaktionsmischung wurde anschließend 1 Stunde bei 0 °C und 1 Stunde bei Raumtemperatur gerührt und dann mit 11.9 g (0.051 Mol) 3-(4-Piperidinyl)-1,3,4,5-tetrahydro-1,3-benzodiazepin-2-on versetzt. Das Gemisch wurde 4 Stunden unter Rückfluß erhitzt und über Nacht bei Raumtemperatur stehen gelassen. Das Reaktionsgemisch wurde am Rotationsverdampfer eingedampft, der Rückstand mit 300 ml wäßriger Natriumhydrogencarbonat-Lösung versetzt und 30 Minuten gerührt. Die wäßrige Lösung wurde abdekantiert, der Rückstand mit 150 ml Ethanol versetzt und unter Rückfluß erhitzt. Nach dem Abkühlen wurde der entstandene weiße Feststoff abgesaugt, mit Ethanol gewaschen und bei 50°C getrocknet. Man erhielt 20.0 g (64% der Theorie) des Produktes mit einem R_{f} Wert von 0.68 (FM D) IR (KBr): 1734, 1680, 1662 (C=O) cm⁻¹

Analog wurden die folgenden Verbindungen der allgemeinen Formel N-B-C hergestellt:

| **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|
| N2 | B2 | OMe | aus N2-H, CDT und H-B2-OMe*HCl und DIEA in THF | 96 | D | 0.76 | ESI: (M-H)⁻ = 620 / 622 / 624 (Br₂); (M+Na)⁺ = 644 / 646 / 648 (Br₂) | 1728, 1664 (C=O) | farblose Kristalle |

### (Referenz)Beispiel A3

### 2-[(3,5-Dibrom-4-fluor-phenyl)methyl]-4-[4-(3,4-dihydro-2(1H)-oxochinazolin-3-yl)-1-piperidinyl]-2-(ethoxycarbonyl)-4-oxobutansäureethylester

Die Mischung aus 4.39 g (0.019 Mol) 3,4-Dihydro-3-(4-piperidinyl)-2(1*H*)-chinazolinon, 9.25 g (0.019 Mol) β,β-Bis-(ethoxycarbonyl)-3,5-dibrom-4-fluor-benzenbutansäure, 6.08 g (0.019 Mol) TBTU, 6.9 ml (0.05 Mol) Triethylamin, 200 ml THF und 70 ml DMF wurde über Nacht bei Zimmertemperatur gerührt. Die Lösemittel wurden im Vakuum entfernt und der Rückstand mit Dichlormethan und 10%-iger wäßriger Zitronensäure-Lösung versetzt. Die organische Phase wurde abgetrennt, mit Natriumhydrogencarbonat-Lösung extrahiert und über Natriumsulfat getrocknet. Nach Entfernen des Trocken- und Lösemittels wurde der Rückstand mit tert-Butylmethylether versetzt und die ausgefallene, feste Substanz abgesaugt. Man erhielt 11.0 g (83% der Theorie) des gewünschten Produktes mit Fp = 167-170°.
IR (KBr): 1734, 1662 (C=O) cm⁻¹
ESI-MS: (M+H)⁺ 696/698/700 (Br₂)

### Beispiel A4

### (R,S)-3,4-Dibrom-phenylalaninethylester

Die Mischung aus 37.40 g (0.140 Mol) *N*-(Diphenylmethylen)-glycinethylester, 55.0 g (0.167 Mol) (3,4-Dibromphenyl)-methylbromid, 6.40 g (0.020 Mol) Tetrabutylammoniumbromid, 57.80 g (0.35 Mol) Kaliumcarbonat Sesquihydrat und 1000 ml Acetonitril wurde 15 Stunden unter Rückfluß gekocht. Der Feststoff wurde abfiltriert, die Mutterlauge im Vakuum eingedampft. Der Rückstand wurde in 400 ml Diethylether aufgenommen und nach Zugabe von 200 ml halbkonzentrierter Salzsäure 1 Stunde bei Zimmertemperatur gerührt. Die organische Phase wurde abgetrennt, die wässerige noch zweimal mit je 50 ml Diethylether gewaschen, dann unter äußerer Kühlung mit Eis mit festem Natriumhydrogencarbonat neutralisiert und mit Essigsäureethylester erschöpfend extrahiert. Die vereinigten Essigsäureethylesterauszüge wurden über Magnesiumsulfat getrocknet, filtriert und im Vakuum eingeengt. Man erhielt das Produkt als hellbraunes Öl. Ausbeute: 33.0 g (67% der Theorie). R_{f} 0.65 (FM D).
IR (KBr): 1734 (C=O) cm⁻¹

Entsprechend wurden die folgenden Verbindungen der allgemeinen Formel N-B-C erhalten:

| **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|
| H | B6 | OEt | aus Ph₂C=NCH₂CO₂Et und 3-Br-4,5-Me₂-C₆H₂-CH₂Br | 60 | | | ESI: (M+H)⁺ = 300/302 (Br) | 1738 (C=O) | farbloses Öl |
| H | B7 | OEt | aus Ph₂C=NCH₂CO₂Et und 3,5-Br₂-4-Me-C₆H₂-CH₂Br | 60 | P | 0.75 | | 1738 (C=O) | farbloses Öl |
| H | B4 | OEt | aus Ph₂C=NCH₂CO₂Et und 3,5-Cl₂-4-Me-C₆H₂-CH₂Br | 70 | B | 0.73 | ESI: (M+H)⁺ 276/278/280 (Cl₂) | 1728 (C=O) | farblose Kristalle, Fp. 44-46 |
| H | B8 | OEt | aus Ph₂C=NCH₂CO₂Et und 3-Cl-4-Me-C₆H₃-CH₂Cl | 83 | O | 0.46 | | 1736 (C=O) | |

### Beispiel A5

### (R,S)-3,4-Difluorphenylalaninmethylester-hydrochlorid

Zu der Suspension von 0.5 g (2.485 mMol) 3,4-Difluorphenylalanin in 10 ml Methanol gab man 4.0 ml gesättigte methanolische Chlorwasserstoff-Lösung und rührte die Mischung 4 Stunden bei Zimmertemperatur. Man dampfte im Vakuum ein, gab zum Rückstand abermals 10 ml Methanol und destillierte das Lösemittel erneut im Vakuum ab. Man erhielt 0.6 g (96% der Theorie) an farblosen Kristallen vom R_{f} 0.7 (FM Dichlormethan).
ESI-MS: (M+H)⁺ = 216

### Beispiel A6

### β,β-Bis-(ethoxycarbonyl)-3,5-dibrom-4-fluor-benzen-butansäure

Zu der eisgekühlten Lösung von 13.1 g (0.037 Mol) β,β-Bis-(ethoxycarbonyl)-3,5-dibrom-4-fluor-benzenbutansäure-1,1-dimethylethylester in 450 ml Dichlormethan tropfte man 70 ml Trifluoressigsäure, entfernte die Kühlung, rührte das Gemisch über Nacht bei Raumtemperatur und engte es anschließend im Vakuum ein. Der Rückstand wurde zweimal durch Koevaporation mit Petrolether getrocknet, mit Petrolether verrieben, abgenutscht und im Vakuum getrocknet. Man erhielt 9.3 g (79% der Theorie) an farblosen Kristallen:
IR (KBr): 1707 (C=O) cm⁻¹
ESI-MS : (M-H)⁻ = 481/483/485 (Br₂)

Entsprechend wurden die folgenden Verbindungen der allgemeinen Formel N-B-C erhalten:

| **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|
| HO | B15[α-CO2Et] | OEt | aus (H₃C)₃CO₂C-B15[α-CO₂Et]-OEt und TFA in CH₂Cl₂ | 81 | V | 0.1 | | 1709 (C=O) | |
| HO | B16[α-CO2Et] | OEt | aus (H₃C)₃CO₂C-B16[α-CO₂Et]-OEt und TFA in CH₂Cl₂ | 100 | | | | 1738 (C=O) | farbloses viskoses Öl |
| HO | B20[α-CO2Et] | OEt | aus (H₃C)₃CO₂C-B20[α-CO₂Et]-OEt und TFA in CH₂Cl₂ | 77 | V | 0.24 | | 3321 (OH); 1714 (C=O); 1161, 1124 (CF₃) | farblose Kristalle |
| HO | B22[α-CO2Et] | OEt | aus (H₃C)₃CO₂C-B22[α-CO₂Et]-OEt und TFA in CH₂Cl₂ | 69 | W | 0.21 | | 1736 (C=O) | farblose Kristalle |
| HO | B25[α-CO2Et] | OEt | aus (H₃C)₃CO₂C-B25[α-CO₂Et)-OEt und TFA in CH₂Cl₂ | 72 | | | | 1730, 1711 (C=O) | farbloses viskoses Öl |
| HO | B27[α-CO2Et] | OEt | aus (H₃C)₃CO₂C-B₂₇[α-CO₂Et]-OEt und TFA in CH₂Cl₂ | 93 | | | | 1736 (C=O) | |
| HO | B24[α-CO2Et] | OEt | aus (H₃C)₃CO₂C-B24[α-CO₂Et]-OEt und TFA in CH₂Cl₂ | 68 | X | 0.28 | | 1709 (C=O) | farblose Kristalle |
| HO | B19[α-CO2Et] | OEt | aus (H₃C)₃CO₂C-B19[α-CO₂Et]-OEt und TFA in CH₂Cl₂ | 46 | | | | | |
| HO | B30[α-CO2Et] | OEt | aus (H₃C)₃CO₂C-B30[α-CO₂Et]-OEt und TFA in CH₂Cl₂ | 81 | | | ESI: (M-H)⁻ = 463/465/467 (Br₂) | | farblose Kristalle |
| HO | B24[α-CO2Et] | OEt | aus (H₃C)₃CO₂C-B24[α-CO₂Et]-OEt und TFA in CH₂Cl₂ | 54 | | | ESI: (M-H)⁻ = 375/377/379 (Cl₂) | | farblose Kristalle |

### Beispiel A7

### 3,5-Dibrom-4-fluor-β,β-bis-(ethoxycarbonyl)-benzenbutansäure-1,1-dimethylethylester

Zu der Lösung von 6.69 g (0.024 Mol) [(1,1-Dimethylethoxy-carbonyl)methyl]-malonsäurediethylester in 170 ml wasserfreiem Tetrahydrofuran gab man unter äußerer Kühlung mit Eiswasser 0.64 g (0.0266 Mol) 95%-iges Natriumhydrid. Nach einstündigem Rühren tropfte man unter Einhaltung einer Reaktionstemperatur von 0 bis +5 °C die Lösung von 8.35 g (0.024 Mol) 3,5-Dibrom-4-fluorbenzylbromid in 30 ml Tetrahydrofuran zu und ließ den Ansatz danach innerhalb von 14 Stunden auf Zimmertemperatur erwärmen. Das Reaktionsgemisch wurde im Vakuum vom Lösemittel befreit, der Rückstand mit 200 ml 10%-iger Zitronensäure versetzt und mit *tert*.-Butylmethylether erschöpfend exrahiert. Die vereinigten Extrakte ergaben nach üblicher Aufarbeitung 13.1 g (100% der Theorie) eines farblosen Öls vom R_{f} = 0.14 (FM Y), das ohne Reinigung in der folgenden Stufe eingesetzt wurde.
IR (KBr): 1732 (C=O) cm⁻¹
ESI-MS: (M+Na)⁺ = 561/563/565 (Br₂)

Analog wurden die folgenden Verbindungen der allgemeinen Formel N-B-C hergestellt:

| **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|
| Me₃CO | B15[α-CO2Et] | OEt | aus (H₃C)₃COCO-CH₂C(CO₂Et)₂, 3,4,5-Me₃-C₆H₂CH₂Br und NaH in THF | 100 | V | 0.6 | | | farbloses Öl |
| Me₃CO | B16[α-CO2Et] | OEt | aus (H₃C)₃COCO-CH₂C(CO₂Et)₂, 3Br-4,5-Me₂-C₆H₂CH₂Br und NaH in THF | 67 | CH₂Cl₂ | 0.71 | | 1736 (C=O) | farbloses Öl |
| Me₃CO | B20[α-CO2Et] | OEt | aus (H₃C)₃COCO-CH₂C(CO₂Et)₂, 2,4-(CF₃)₂-C₆H₃CH₂Br und NaH in THF | 100 | V | 0.72 | kein M⁺; (M-C₄H₈)⁺ = 444 | 1736 (C=O) | |
| Me₃CO | B22[α-CO2Et] | OEt | aus (H₃C)₃COCO-CH₂C(CO₂Et)₂, 3,4,5Br₃-C₆H₂CH₂Br und NaH in THF | 91 | W | 0.78 | | 1734 (C=O) | farbloses Öl |
| Me₃CO | B25[α-CO2Et] | OEt | aus (H₃C)₃COCO-CH₂C(CO₂Et)₂, 4-Br-3,5Cl₂-C₆H₂CH₂Br und NaH in THF | 100 | Y | 0.75 | | | farbloses viskoses Öl |
| Me₃CO | B27[α-CO2Et] | OEt | aus (H₃C)₃COCO-CH₂C(CO₂Et)₂, 3,4-(CH₂)₂O-C₆H₃CH₂Br und NaH in THF | 58 | Y | 0.31 | M⁺ = 406 | 1734 (C=O) | |
| Me₃CO | B29[α-CO2Et] | OEt | aus (H₃C)₃COCO-CH₂C(CO₂Et)₂, 2,3Cl₂-C₆H₃CH₂Cl und NaH in THF | 89 | X | 0.49 | | 1736 (C=O) | farbloses Öl |
| Me₃CO | B19[α-CO2Et] | OEt | aus (H₃C)₃COCO-CH₂C(CO₂Et)₂, 4NH₂-3,5Cl₂-C₆H₂CH₂Br und NaH in THF | 88 | | | | | farbloses Öl |

### Beispiel A8

### 3,4-Dimethoxy-β-(methoxycarbonyl)-benzenbutansäure

Die Lösung von 58.0 g (0.205 Mol) 4-[(3,4-Dimethoxyphenyl]-3-(methoxycarbonyl)-3-butensäure in 500 ml Methanol wurde in Gegenwart von 3.0 g 10%-igem Platin/- Aktivkohle bis zur Beendigung der Wasserstoffaufnahme bei 5 bar Wasserstoff hydriert. Nach der üblichen Aufarbeitung erhielt man 26.0 g (46% der Theorie) an farblosen Kristallen mit Fp = 104-107°C

Entsprechend wurde die folgenden Verbindungen der allgemeinen Formel N-B-C erhalten:

| **N** | **B** | **C** | **Anmerkungen** | **% Ausb.** | **FM** | **R_{f}** | **Fp. [°C]** |
|---|---|---|---|---|---|---|---|
| HO | B26 | OMe | aus 4-(2-Naphthyl)-3-(methoxycarbonyl)-3-butensäure, H₂ und Pd-C in MeOH | | X | 0.85 | |

### Beispiel A9

### 4-[(3,4-Dimethoxy-phenyl]-3-(methoxycarbonyl)-3-buterisäure

Zu einer frisch bereiteten Lösung von 4.6 g (0.2 Mol) Natrium in 250 ml wasserfreiem Methanol gab man 26.6 ml (0.2 Mol) Bernsteinsäuredimethylester und tropfte nach einstündigem Rühren bei Zimmertemperatur die Lösung von 33.3 g (0.2 Mol) 3,4-Dimethoxybenzaldehyd in 100 ml wasserfreiem Methanol zu. Danach kochte man 6 Stunden unter Rückfluß, entfernte das Methanol im Vakuum und hielt den verbleibenden Sumpf 30 Minuten bei einer Reaktionstemperaturvon 80°C. Der erhaltene zähe Brei wurde in 500 ml Wasser aufgenommen, mit 20%-iger wässeriger Zitronensäure-Lösung angesäuert und das anfallende Gemisch mit Essigsäureethylester erschöpfend extrahiert. Die vereinigten Essigesterextrakte wurden ihrerseits fünfmal mit 5%-iger wässeriger Ammoniak-Lösung ausgezogen. Die ammoniakalischen Extrakte wurden vorsichtig mit 20%-iger wässeriger Zitronensäure-Lösung angesäuert und anschließend mit Essigester erschöpfend extrahiert. Diese Extrakte wurden mit Wasser gewaschen, über Natriumsulfat getrocknet und vom Lösemittel im Vakuum befreit. Das Rohprodukt (Ausbeute quantitativ) wurde ohne Reinigung weiter umgesetzt.

Entsprechend wurden die folgenden Verbindungen der allgemeinen Formel N-B-C erhalten:

| **N** | **B** | **C** | **Anmerkungen** | **% Ausb.** | **FM** | **R_{f}** |
|---|---|---|---|---|---|---|
| 4-(2-Naphthyl)-3-(methoxycarbonyl)-3- butensäure | | | aus 2-Naphthaldehyd, Bernsteinsäure-dimethylester und NaOMe in MeOH | 65 | X | 0.8 |

### Beispiel A10

### [1,4']Bipiperidinyl-4-essigäuremethylester

Die Lösung von 0.669 g (2.024 mMol) 1'-Phenylmethyl-[1,4']bipiperidinyl-4-essigsäuremethylester in 20 ml Methanol wurde nach Zugabe von 100 mg 10%-iger Palladiumkohle bei einem Druck von 5 bar bis zur Beendigung der Wasserstoffaufnahme hydriert. Der Katalysator wurde abfiltriert, das Filtrat vom Lösemittel befreit, der Rückstand in 20 ml THF aufgenommen, die entstandene Lösung fitriert und erneut eingedampft. Der Rückstand wurde ohne weitere Reinigung verwendet. Farbloses Öl. Ausbeute: 490 mg (100% der Theorie).

| | |
|---|---|
| ESI-MS: | (M+H)⁺ = 241 |
| | (M+Na)⁺ = 253 |

Entsprechend wurden die folgenden Verbindungen der allgemeinen Formel N-B-C erhalten:

| **N** | **B** | **C** | **Anmerkungen** | **% Ausb.** | **FM** | **R_{f}** | **MS** | **Fp. [°C]** |
|---|---|---|---|---|---|---|---|---|
| H | - | C5 | aus PhCH₂-C5, H₂ und Pd/C in MeOH | 100 | G | 0.22 | ESI: (M+H)⁺ = 241; (M+Na)⁺ = 253 | farbloses Öl |
| H | - | C12 | aus PhCH₂-C12, H₂ und Pd/C in EtOH | 98 | D | 0.17 | ESI: (M+H)⁺ = 284 | farblose Kristalle |
| H | - | C9 | aus PhCH₂-C9, H₂ und Pd/C in EtOH | 78 | O | 0.1 | | farbloses Öl |
| H | - | C3 | aus PhCH₂-C3, H₂ und Pd/C in MeOH | 99 | | | ESI: (M+H)⁺ = 284; (M+Na)⁺ = 306 | farbloses Öl |
| H | - | C1 | aus PhCH₂-Cl, H₂ und Pd/C in EtOH | 97 | M | 0.38 | ESI: (M+H)⁺ = 256 | |
| H | - | C14 | aus PhCH₂-C14, H₂ und Pd/C in MeOH | 79 | G | 0.14 | ESI: (M+H)⁺ = 213 | farblose Kristalle |
| H | - | C16 | aus PhCH₂-C16, H₂ und Pd/C in MeOH | 67 | G | 0.16 | ESI: (M+H)⁺ = 213 | farblose Kristalle |
| H | - | C19 | aus PhCH₂-C19, H₂ und Pd/C in MeOH | 100 | G | 0.20 | ESI: (M+H)⁺ = 227 | farbloses Öl |
| H | - | C22 | aus PhCH₂-C22, H₂ und Pd/C in MeOH | 100 | C | 0.06 | ESI: (M+H)⁺ = 227 | farblose Kristalle |
| H | - | C26 | aus PhCH₂-C26, H₂ und Pd/C in MeOH | 100 | | | | farblose Kristalle |
| H | - | C28 | aus 4-[(1-Phenylmethyl)-1,2,3,6-tetrahydro-4-pyrididinyl]-benzoesäuremethyl-ester, H₂ und Pd/C in MeOH | 70 | S | 0.4 | | farblose Kristalle |
| H | - | C18 | Acetat, aus PhCH₂-C18, H₂ und Pd/C in MeOH | 88 | G | 0.20 | ESI: (M+H)⁺ = 227 | farbloses viskoses Öl |
| H | - | C7 | aus PhCH₂-C7, H₂ und Pd/C in EtOH | 92 | O | 0.15 | ESI: (M+H)⁺ = 241; (M+Na)⁺ = 263 | farbloses Öl |
| H | - | C50 | aus PhCH₂-C50, H₂ und Pd(OH)₂ (Pearlman's catalyst) in EtOH | 100 | KK | 0.21 | ESI: (M+H)⁺ = 256 | farbloses viskoses Öl |
| 4-Methyl-2-piperazin-carbonsäure-ethylester | | | aus 1-(Phenylmethyl)-4-methyl-2-piperazincarbonsäure-ethylester, H₂ und Pd(OH)₂ (Pearlman's catalyst) in EtOH | 99 | | | | farbloses Öl |
| H | - | C46 | aus PhCH₂-C46, H₂ und Pd(OH)₂ (Pearlman's catalyst) in EtOH | 100 | DD | 0.24 | ESI: (M+H)⁺ 256 | = farbloses viskoses Öl |
| H | - | C45 | aus PhCH₂-C45, H₂ und Pd(OH)₂ (Pearlman's catalyst) in EtOH | 100 | LL | 0.1 | ESI: (M+H)⁺ = 256 | farbloses Öl |
| 2-Piperazin-carbonsäure-ethylester | | | aus 1,4-Bis-(phenylmethyl)-2-piperazincarbonsäure-ethylester, H₂ und 10proz. Pd/C in EtOH | 100 | MM | 0.2 | ESI: (M+H)⁺ = 159 | |

### Beispiel A11

### 1'-(Phenylmethyl)-[1,4']bipiperidinyl-4-essigsäuremethylester

Zu der Mischung aus 4.549 ml (24.54 mMol) 1-(Phenylmethyl)-4-piperidinon, 4.753 g (24.54 mMol) 4-Piperidinessigsäure-methylester-hydrochlorid und 40 ml THF gab man 4.0 ml Eisessig und 20 g Molekularsieb 3 Å, ließ 2 Stunden bei Zimmertemperatur rühren, kühlte auf 0 °C ab und trug unter Einhaltung dieser Temperatur in kleinen Portionen innerhalb von 8 Stunden insgesamt 6.358 g (30.0 mMol) Natriumtriacetoxyborhydrid ein. Anschließend rührte man noch 16 Stunden bei Zimmertemperatur. Man stellte den Ansatz natriumhydrogencarbonat-alkalisch, extrahierte erschöpfend mit Essigsäureethylester, trocknete die vereinigten Extrakte über Natriumsulfat und chromatographierte den Eindampfrückstand an Kieselgel unter Verwendung von anfangs Dichlormethan/Methanol 30/1, dann 20/1, zuletzt 10/1 als Eluenzien. Aufarbeitung der geeigneten Fraktionen ergab 1.804 g (22% der Theorie) eines leicht beweglichen Öls, das über Nacht zu farblosen Kristallen erstarrte. R_{f} =0.56 (FM B).
ESI-MS: (M+H)⁺ = 331.

Entsprechend wurden die folgenden Verbindungen der allgemeinen Formel N-B-C erhalten:

| **N** | **B** | **C** | **Anmerkungen** | **% Ausb.** | **FM** | **R_{f}** | **MS** | **Fp. [°C]** |
|---|---|---|---|---|---|---|---|---|
| PhCH₂ | - | C7 + C9 | aus 1-(Phenylmethyl)-piperazin, 4-Oxocyclohexancarbon-säureethylester und Na(CN)BH₃/AcOH in MeOH bei pH 5-6; Trennung der beiden Diastereomeren an Kieselgel, FM Dichlormethan / MeOH 30/1 v/v | cis: 14.7 + trans: 13.8 + cis / trans: 5.8 | AA | cis: 0.40; trans: 0.30 | cis: ESI: (M+H)⁺ = 331; (M+Na)⁺ = 353; trans: ESI: (M+H)⁺ = 331 | farblose Flüssigkeiten |
| PhCH₂ | - | C3 | aus 1-(Phenylmethyl)-piperazin, 4-Oxo-1-piperidinessigsäure-1,1-dimethylethylester und Na(CN)BH₃/AcOH in MeOH bei pH 5-6 | 58 | O | 0.67 | ESI: (M+H)⁺ = 374; (M+Na)⁺ = 396 | farblose Kristalle |
| 4-[1-(Phenylmethyl)-4-piperidinyl]-1-(1,1-dimethylethoxycarbonyl) -piperazin | | | aus 1-(Phenylmethyl)-4-piperidinon, 1-(1,1-Dimethylethoxycarbonyl) -piperazin und NaBH(OAc)₃/AcOH in THF | 100 | D | 0.60 | ESI: (M+H)⁺ = 360; (M+Na)⁺ = 382; (2M+Na)⁺ = 741 | farbloses Öl |
| PhCH₂ | - | C14 | aus 1-(Phenylmethyl)-4-piperidinon, L-Prolinmethylester-hydrochlorid und NaBH(OAc)₃/AcOH in THF | 51 | G | 0.50 | ESI: (M+H)⁺ = 303 | farbloses Öl |
| PhCH₂ | - | C16 | aus 1-(Phenylmethyl)-4-piperidinon, D-Prolinmethylester-hydrochlorid und NaBH(OAc)₃/AcOH in THF | 54 | G | 0.50 | ESI: (M+H)⁺ = 303; (M+Na)⁺ = 325 | farbloses Öl |
| PhCH₂ | - | C19 | aus 1-(Phenylmethyl)-4-piperidinon, L-Homoprolinmethylester-hydrochlorid [Bachem] und NaBH(OAc)₃ in CH₂Cl₂ | 51 | G | 0.40 | ESI: (M+H)⁺ = 317; (M+Na)⁺ = 339 | farbloses Öl |
| PhCH₂ | - | C18 | aus 1-(Phenylmethyl)-4-piperidinon, D-Homoprolinmethylester-hydrochlorid [Bachem] und NaBH(OAc)₃ in CH₂Cl₂ | 57 | G | 0.40 | ESI: (M+H)⁺ = 317 | farbloses viskoses Öl |
| PhCH₂ | - | C50 | aus 1-(Phenylmethyl)-4-piperidinon, 4-Methyl-2-piperazincarbonsäure-ethylester und NaBH(OAc)₃ in THF | 22 | DD | 0.84 | ESI: (M+H)⁺ = 346 | |
| PhCH₂ | - | C46 | aus 1-Methyl-4-piperidinon, 1-(Phenylmethyl)-2-piperazincarbonsäure-ethylester-bis-(trifluoracetat) und NaBH(OAc)₃ in THF | 100 | C | 0.53 | ESI: (M+H)⁺ = 346 | farblosesÖl |
| PhCH₃ | - | C45 | aus 1-(Phenylmethyl)-4-piperidinon, 1-Methyl-2-piperazincarbonsäure-ethylester-bis-(trifluoracetat) und NaBH(OAc)₃ in THF | 100 | C | 0.41 | M⁺ = 345 | farbloses Öl |
| Boc | - | C44 | aus 1-Methyl-4-piperidinon, 4-(1,1-Dimethylethoxycarbonyl) -2-piperazincarbonsäure-ethylester-bis-(trifluoracetat) und NaBH(OAc)₃ in THF | 57 | C | 0.46 | ESI: (M+H)⁺ = 356 | farbloses viskoses Öl |

### Beispiel A12

### 4-[1-(Phenylmethyl)-4-piperidinyl]-1-piperazinessigsäure-ethylester

Zu der Suspension von 2.0 g (3.325 mMol) 1-(Phenylmethyl)-4-(1-piperazinyl)-piperidin-tris-(trifluoracetat) in 50 ml Dichlormethan gab man 3.5 ml (19.892 mMol) DIEA und rührte 10 Minuten bei Zimmertemperatur. Dann trug man 0.38 ml (3.365 mMol) Bromessigsäureethylester ein und rührte über Nacht bei Zimmertemperatur. Das Reaktionsgemisch wurde viermal mit je 50 ml Wasser ausgeschüttelt, über Natriumsulfat getrocknet und eingedampft. Man erhielt 0.70 g (61% der Theorie) des gesuchten Produkts vom R_{f} 0.63 (FM D) und
ESI-MS: (M+H)⁺ = 346.

Entsprechend wurde die folgende Verbindung der allgemeinen Formel N-B-C erhalten:

| **N** | **B** | **C** | **Anmerkungen** | **% Ausb.** | **FM** | **R_{f}** | **MS** | **Fp. [°C]** |
|---|---|---|---|---|---|---|---|---|
| PhCH₂ | - | C12 | aus 1-(Phenylmethyl)-4-(1-piperazinyl)-piperidin-tris-(trifluoracetat), Bromessiggäure-1,1-dimethylethylester und K₂CO₃ in CH₃CN | 65 | D | 0.51 | ESI: (M+H)⁺ = 374; (M+Na)⁺ = 396 | farblose Kristalle |

### Beispiel A13

### 1-(Phenylmethyl)-4-(1-piperazinyl)-piperidin-tris-(trifluoracetat)

Das Gemisch aus 77.6 g (0.216 Mol) 4-[1-(Phenylmethyl)-4-piperidinyl]-1-(1,1-dimethylethoxycarbonyl)-piperazin, 150 ml (1.941 Mol) Trifluoressigsäure und 450 ml Dichlormethan wurde 1 Stunde unter Rückfluß gekocht und dann 2 Stunden bei Zimmertemperatur gerührt. Das Lösemittel wurde abdestilliert, der Rückstand mit Diethylether verrieben, abgenutscht und an der Luft getrocknet. Man erhielt 119.0 g (92% der Theorie) an farblosen Kristallen vom R_{f} 0.20 (FM D) und
ESI-MS: (M+H)⁺ = 260

Entsprechend wurden die folgenden Verbindungen der allgemeinen Formel N-B-C erhalten:

| **N** | **B** | **C** | **Anmerkungen** | **% Ausb.** | **FM** | **R_{f}** | **MS** | **Fp. [°C]** |
|---|---|---|---|---|---|---|---|---|
| H | - | C29 | aus 4-[[1-(1,1-Dimethylethoxycarbonyl)-4-piperidinyl]methyl]-benzoesäureethylester und TFA in CH₂Cl₂ | 89 | BB | 0.70 | | farblose Kristalle |
| H | - | C44 | aus 4-(1,1-Dimethylethoxycarbonyl)-1-(1-methyl-4-piperidihyl)-2-piperazincarbonsäure-ethylester und TFA in CH₂Cl₂ | 100 | DD | 0.11 | M⁺ = 255 | farbloses viskoses Öl |
| 1-(Phenylmethyl) -2-piperazin-carbonsäure-ethylester-bis-(trifluoracetat) | | | aus 4-(1,1-Dimethylethoxycarbonyl)-1-(phenylmethyl)-2-piperazincarbonsäure-ethylester und TFA in CH₂Cl₂ | 100 | AcOEt | 0.00 | ESI: (M+H)⁺ = 249 | farbloses Öl |
| 1-Methyl-2-piperazin-carbonsäure-ethylester-bis-(trifluoracetat) | | | aus 4-(1,1-Dimethylethoxycarbonyl)-1-methyl-2-piperazincarbonsäure-ethylester und TFA in CH₂Cl₂ | 100 | DD | 0.16 | ESI: (M+H)⁺ = 173 | farbloses viskoses Öl |

### Beispiel A14

### 1'-(Phenylmethyl)-[1,4']bipiperidinyl-4'-carbonsäuremethylester

Zu der Lösung von 1.0 g (3.307 mMol) 1'-(Phenylmethyl)-[1,4']bipiperidinyl-4'-carbonsäure in 30 ml DMF gab man 1.124 g (3.5 mMol) TBTU und 1.0 ml (7.175 mMol) Triethylamin, rührte 20 Minuten bei Zimmertemperatur, fügte dann 20 ml Methanol zu und rührte weitere 3 Stunden bei Raumtemperatur. Die Mischung wurde eingedampft, der Rückstand in 50 ml Essigsäureethylester aufgenommen und filtriert. Das Filtrat wurde eingeengt, der Rückstand säulenchromatogaphisch an Kieselgel unter Verwendung von anfangs Ethylacetat, dann von Ethylacetat im Gemisch mit bis zu 5% Methanol/konz. Ammoniak (9/1 v/v) zum Eluieren gereinigt. Man erhielt 0.231 g (22% der Theorie) an farblosen Kristallen vom Fp. 84.7 °C und
Rf 0.73 (FM F).
ESI-MS: (M+H)⁺ = 317

### Beispiel A15

### 3-(4-Piperidinyl)-benzoesäuremethylester-hydrochlorid

Die Mischung aus 500 mg (2.069 mMol) 3-(4-Piperidinyl)-benzoesäure-hydrochlorid und 10 ml gesättigter methanolischer Chlorwasserstoff-Lösung wurde über Nacht bei Zimmertemperatur gerührt. Das reaktionsgemisch wurde im Vakuum eingedampft, der Rückstand mit 3 ml Isopropanol verrührt, abgenutscht, mit Diethylether gewaschen und bei 60 °C im Umlufttrockenschrank getrocknet. Man erhielt 390 mg (74% der Theorie) an farblosen Kristallen vom R_{f} 0.34 (FM D).

| | |
|---|---|
| IR (KBr): | 1728 (C=O) cm⁻¹ |
| ESI-MS: | (M+H)⁺ = 220; |
| | (M+Cl+HCl)⁻ = 290/292/294 (Cl₂) |

Entsprechend wurden folgende Ester der allgemeinen Formel N-B-C erhalten:

| **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|
| H | - | C31 | Dihydrochlorid; aus H-C38 [BAYER], MeOH und HCl | 76 | D | 0.58 | ESI: (M+H)⁺ = 289; (M+Cl+HCl)⁻ = 359/361/363 (Cl₂) | 1722 (C=O) | farblose Kristalle |
| PhCH₂ | - | C41 | aus PhCH₂-C43, MeOH und HCl | 52 | D | 0.88 | ESI: (M+H)⁺ = 318; (M+Na)⁺ = 340; (2M+Na)⁺ = 657 | | |
| 2-Aminothiazol-5- carbonsäuremethyl- ester-hydrochlorid | | | aus 2-Aminothiazol-5-carbonsäure, MeOH und HCl | 100 | D | 0.59 | ESI: (M+H)⁺ = 159; (M-H)⁻ = 157 | | |
| 4-[1-(Phenylmethyl)-1,2,3,6-tetrahydro-4-pyridinyl]-benzoesäure-methylester | | | aus 4-[1-(Phenylmethyl)-1,2,3,6-tetrahydro-4-pyridinyl]-benzoesäure, MeOH und HCl | 85 | | | ESI: (M+H)⁺ = 308 | 1707 (C=O) | |

### Beispiel A16

### 1'-(Phenylmethyl)-[1,4']bipiperidinyl-4'-carbonsäure

In 15 ml konz. Schwefelsäure wurden in kleinen Portionen insgesamt 5.0 g (17.642 mMol) 1'-(Phenylmethyl)-[1,4']bipiperidinyl-4'-carbonitril eingetragen. Nachdem das Nitril in Lösung gegangen war, wurde weitere 3 Stunden bei Zimmertemperatur gerührt, dann 10 ml Wasser zugegeben und die Mischung 15 Stunden unter Rückfluß gekocht. Der erkaltete Ansatz wurde in 50 ml Eiswasser eingerührt und mit konz. Ammoniak auf pH 7 gebracht. Der ausgefallene Niederschlag wurde abgenutscht, mit wenig Wasser gewaschen, mit 10 ml Dichlormethan verrührt, erneut abgenutscht, dann im Vakuum getrocknet. Man erhielt 1.56 g (29% der Theorie) an farblosen Kristallen vom Rf 0.0 (FM DD).
ESI-MS: (M+H) = 303

### Beispiel A17

### 3-(1-Piperazinyl)-benzoesäureethylester

Zu der Lösung von 18.5 g (0.055 Mol) 3-[4-(Phenylmethoxycarbonyl)-1-piperazinyl]-berizoesäureethylester in 30 ml Eisessig tropfte man bei Zimmertemperatur 30 ml einer gesättigten Lösung von Bromwasserstoff in Eisessig und rührte weitere 4 Stunden bei Zimmertemperatur. Der Mischung wurden 300 ml Diethylether zugesetzt, der entstandene Niederschlag anschließend abgenutscht, mit Diethylether gründlich gewaschen und an der Luft getrocknet. Ausbeute 17.8 g (82% der Theorie). Farblose Kristalle vom Fp. 226°C (Z) und R_{f} 0.24 (FM EE). C₁₃H₁₈N₂O₂*2 HBr (396.13)

| | | | | |
|---|---|---|---|---|
| Ber.: | C39.42 | H5.09 | N7.07 | Br40.34 |
| Gef.: | 39.27 | 5.06 | 7.15 | 40.35 |

### Beispiel A18

### 3-[4-(Phenylmethoxycarbonyl)-1-piperazinyl]-benzoesäure-ethylester

Zu der Lösung von 26.0 g (0.08 Mol) 3-[4-(Phenylmethyl)-1-piperazinyl]-benzoesäureethylester in 260 ml Dichlormethan gab man im Abstand von 16 Stunden zweimal je 15.0 g (zusammen 0.176 Mol) Chlorkohlensäurebenzylester und rührte insgesamt 32 Stunden bei Zimmertemperatur. Das Lösemittel wurde im Vakuum entfernt, der Rückstand säulenchromatographisch an Kieselgel unter Verwendung von Dichlormethan zum Eluieren gereinigt. Man erhielt 18.8 g (70% der Theorie) eines farblosen Öls vom R_{f} 0.67 (FM FF).

### Beispiel A19

### 3-[4-(Phenylmethyl)-1-piperazinyl]-benzoesäureethylester-hydroiodid

Die Mischung aus 53.6 g (0.2 Mol) *N*,*N*-Bis.(2-chlorethyl)-benzenmethanamin-hydrochlorid, 40.2 g (0.2 Mol) 3-Aminobenzoesäureethylester-hydrochlorid, 30.0 g (0.2 Mol) Natriumiodid, 20.0 g Natriumcarbonat und 1 l n-Propanol wurde 2 Stunden lang unter Rückfluß gekocht. Man kühlte auf 80°C ab, gab langsam weitere 15 g Natriumcarbonat zu und kochte abermals 2 Stunden unter Rückfluß. Nach Abkühlen auf 80°C wurde das restliche Natriumcarbonat von einer Gesamtmenge von 53.0 g (0.5 Mol) zugesetzt und nochmals 2 Stunden bei Rückflußtemperatur gehalten. Man ließ erkalten, filtrierte von den unlöslichen Salzen ab und engte das Filtrat im Vakuum ein. Der Rückstand wurde in 200 ml Dichlormethan aufgenommen, die Dichlormethan-Lösung zweimal mit je 50 ml 1N Salzsäure gewaschen, dann eingedampft. Der verbliebene Rückstand ergab nach dem Umkristallisieren aus Ethanol 43.0 g (48% der Theorie) an farblosen Kristallen vom Fp. 180-182 °C und
R_{f} = 0.62 (FM GG).

### Beispiel A20

### 4-[1-(Phenylmethyl)-1,2,3,6-tetrahydro-4-pyridinyl]-benzoesäure

Zu der Lösung von 13.13 g (0.040 Mol) 4-(4-Bromphenyl)-1-(phenylmiethyl)-1,2,3,6-tetrahydropyridin in 190 ml wasserfreiem THF tropfte man unter Argon-Atmosphäre und Einhaltung einer Reaktionstemperatur von -70 bis -60 °C 25.0 ml (0.04 Mol) einer 1.6-molaren Lösung von *n*-Butyllithium in *n*-Hexan. Nach 30 Minuten bei -60°C goß man den Ansatz unter gutem Rühren auf 500 g fein zerstoßenes Trockeneis und ließ dann die Mischung über Nacht auf Zimmertemperatur kommen. Man verdünnte mit 300 ml Diethylether und extrahierte dann mit zweimal je 100 ml Wasser. Unter äußerer Kühlung wurden die vereinigten wässerigen Auszüge mit 2N Salzsäure auf pH 7.5 gebracht. Der entstandene Niederschlag wurde abgenutscht, mit 50 ml heißem Methanol verrührt und nach dem Erkalten erneut abgenutscht. Man erhielt nach dem Trocknen im Exsikkator 8.3 g (71% der Theorie) an farblosen Kristallen vom R_{f} 0.5 (FM HH).

| | |
|---|---|
| ESI-MS: | (M+H)⁺ = 294 |
| | (M-H)⁻ = 292 |

### Beispiel A21

### 4-(4-Bromphenyl)-1-(phenylmethyl)-4-piperidinol

Zu der Lösung von 23.591 g (0.10 Mol) 1,4-Dibrombenzol in 250 ml wasserfreiem THF tropfte man unter Einhaltung einer Reaktionstemperatur von -60 bis -50 °C 62.5 ml (0.1 Mol) einer 1.6-molaren Lösung von *n*-Butyllithium in *n*-Hexan. Man rührte weitere 20 Minuten bei der genannten Temperatur, bevor man die Lösung von 18.926 g (0.10 Mol) 1-(Phenylmethyl)-4-piperidinon in 50 ml wasserfreiem THF zutropfte. Man ließ auf Zimmertemperatur erwärmen, rührte noch über Nacht bei dieser Temperatur, trug den Ansatz dann in Eiswasser ein und extrahierte erschöpfend mit Essigsäureethylester. Die vereinigten Essigesterextrakte wurden mit Wasser und gesättigter Kochsalzlösung gewaschen, über Natriumsulfat getrocknet und im Vakuum eingedampft. Der Rückstand wurde aus Diisopropylether umkristallisiert. Man erhielt 23.1 g (67% der Theorie) an farblosen Kristallen vom R_{f} 0.4 (FM BB).

### Beispiel A22

### 4-[[1-(1,1-Dimethylethoxycarbonyl)-4-piperidinyl]methyl]-benzoesäureethylester

Die Lösung von 38.7 g (0.112 Mol) 1-(1,1-Dimethylethoxycarbonyl)-4-[4-(ethoxycarbonyl)-phenylmethylen]-piperidin in 350 ml Essigsäureethylester wurde in Gegenwart von 4.82 g 10%-igem Pallaium auf Kohle bis zur Beendigung der Wasserstoffaufnahme bei Zimmertemperatur und einem Druck von 5 bar hydriert. Übliche Aufarbeitung ergab 35.8 g (92% der Theorie) eines farblosen Öls, das ohne weitere Reinigung verwendet wurde.

### Beispiel A23

### 1-(1,1-Dimethylethoxycarbonyl)-4-[4-(ethoxycarbonyl)-phenylmethylen]-piperidin

Zu der Lösung von 19.2 ml (0.135 Mol) Diisopropylamin in 400 ml wasserfreiem THF tropfte man unter Verwendung von Argon als Schutzgas und unter Einhaltung einer Reaktionstemperatur von -20 bis -10 °C 85.0 ml (0.136 Mol) einer 1.6-molaren Lösung von *n*-Butyllithium in *n*-Hexan. Man hielt noch 20 Minuten die genannte Temperatur und tropfte dann die Lösung von 39.35 g (0.131 Mol) [4-(Ethoxycarbonyl)phenyl]-methanphosphonsäure-diethylester in 100 ml THF zu. Man rührte weitere 20 Minuten bei einer Temperatur zwischen -20 und -10 °C, tropfte dann die Lösung aus 28.1 g (0.131 Mol) 1-(1,1-Dimethylethoxy-carbonyl)-4-piperidinon in 100 ml THF ein und ließ über Nacht auf Zimmertemperatur erwärmen. Der Ansatz wurde in Eiswasser eingerührt, die entstandene Mischung mit Essigsäureethylester erschöpfend extrahiert, die vereinigten Extrakte mit gesättigter wässeriger NaCl-Lösung gewaschen, über Natriumsulfat getrocknet und vom Lösemittel befreit. Der Rückstand wurde säulenchromatographisch an Kieselgel und unter Verwendung' von Petrolether/Essigsäureethylester 7/1 v/v zum Eluieren gereinigt. Man erhielt 38.7 g (86% der Theorie) eines farblosen Öls, das in Gegenwart von Petrolether zu farblosen Kristallen erstarrte.

### Beispiel A24

### [4-(Ethoxycarbonyl)phenyl]-methanphosphonsäurediethylester

In einer Rührapparatur wurden 55 ml (0.316 Mol) Triethylphosphit vorgelegt und auf eine Innentemperatur von 90°C vorgeheizt. Hierzu wurde langsam und in kleinen Portionen die Suspension von 60.0 g (0.247 Mol) 4-(Brommethyl)-benzoesäureethylester in 100 ml Dichlormethan gegeben, wobei das entstandene Ethylbromid und das verdampfende Dichlormethan laufend abdestilliert wurden. Nachdem die Menge des entstandenen Ethylbromids deutlich zurückgegangen war, wurde die Reaktionstemperatur langsam auf 140°C gesteigert und diese Temperatur bis zur Beendigung der Ethylbromidbildung (ca. 2 Stunden) gehalten. Das überschüssige Triethylphosphit wurde im Vakuum entfernt, der Rückstand in wenig Essigsäureethylester suspendiert und an Kieselgel unter Verwendung von Essigsäureethylester/Petrolether (Gradient 1/1 → 1/0 v/v) zum Eluieren säulenchromatographisch gereinigt. Nach üblicher Aufarbeitung erhielt man 56.3 g (76% der Theorie) der obigen Titelverbindung in Form eines farblosen Öls.

### Beispiel A25

### 4-[2-(4-Piperidinyl)ethyl]-benzoesäureethylester

Die Lösung von 22.0 g (0.076 Mol) 4=[2-(4-Pyridinyl)vinyl]-benzoesäureethylester-hydrochlorid in 800 ml Ethanol wurde in Gegenwart von 2 g Platin(IV)-oxid bei 3.8 bar Wasserstoffdruck 8 Stunden lang hydriert. Katalysator und Lösemittel wurden entfernt, der Rückstand in 5%-iger Salzsäure aufgenommen und zweimal mit je 50 ml Diethylether extrahiert. Die wässerige Phase wurde sodaalkalisch gestellt und mit Essigsäureethylester erschöpfend ausgeschüttelt. Die vereinigten Ethylacetatauszüge wurden mit gesättigter Kochsalz-Lösung gewaschen, über Natriumsulfat getrocknet und eingedampft. Das erhaltene ölige Produkt (17.0 g, 86% der Theorie) wurde ohne weitere Reinigung verwendet.

### Beispiel A26

### (E)-[2-(4-Pyridinyl)vinyl]-benzoesäureethylester-hydrochlorid

Zu der Suspension von 1.87 g (78 mMol) Natriumhydrid in 150 ml THF tropfte man unter Einhaltung einer Reaktionstemperatur von -10 bis 0°C die Lösung von 9.1 g (85.0 mMol) 4-Pyridin-carboxaldehyd und 25.0 g (83.3 mMol) [4-(Ethoxycarbonyl)-phenyl]-methanphosphonsäurediethylester in 150 ml THF. Die Mischung wurde 35 Stunden unter Stickstoffatmosphäre gerührt. Dann wurde zwischen Wasser und Diethylether verteilt, die etherische Phase über Natriumsulfat getrocknet, auf ein Volumen von ca. 200 ml eingedampft und mit etherischer Chlorwasserstoff-Lösung bis zur Beendigung der Fällungsreaktion versetzt. Die entstandenen farblosen Kristalle wurden abgenutscht, mit Diethylether gewaschen und an der Luft getrocknet. Ausbeute: 22.0 g (87% der Theorie). Fp. 215-225 °C.

### Beispiel A27

### 2-(1-Piperazinyl)-thiazol-5-carbonsäuremethylester

Zu der Lösung von 4.2 g (23.647 mMol) 2-Chlorthiazol-5-carbonsäuremethylester in 5 ml Ethanol gab man 10.0 g (116.09 mMol) wasserfreies Piperazin und kochte 3 Stunden unter Rückfluß. Das Reaktionsgemisch wurde mit gesättigter wässeriger Natriumhydrogencarbonat-Lösung versetzt und mit Essigsäureethylester erschöpfend extrahiert. Die vereinigten organischen Auszüge wurden gründlich mit Wasser gewaschen, über Natriumsulfat getrocknet und im Vakuum eingedampft. Man erhielt 1.8 g (34% der Theorie) an farblosen Kristallen vom R_{f} 0.44 (FM D).

### Beispiel A28

### 2-Chlorthiazol-5-carbonsäuremethylester

Zu der Suspension von 14.0 g (71.927 mMol) 2-Aminothiazol-5-carbonsäuremethylester-hydrochlorid in 8 ml konz. Salzsäure gab man 20 g gestoßenes Eis und tropfte unter äußerer Kühlung die Lösung von 5.0 g (72.464 mMol) Natriumnitrit in 30 ml Wasser ein, wobei die Reaktionstemperatur stets unter 0 °C gehalten wurde. Nach 30 Minuten gab man 7.2 g (72.735 mMol) Kupfer(I)-chlorid zu, rührte noch 1 Stunde unter Kühlung und ließ in den folgenden 1½ Stunden langsam auf Zimmertemperatur kommen. Das Gemisch wurde mit Diethylether erschöpfend extrahiert, die vereinigten Extrakte wurden mit gesättigter Kochsalzlösung gewaschen, über Natriumsulfat getrocknet und eingeengt. Man erhielt 4.3 g (34% der Theorie) eines farblosen Öls vom Rf = 0.94 (FM D), das ohne weitere Reinigung in den nachfolgenden Stufen verwendet wurde.
MS: M⁺ = 177/179 (CI)

### Beispiel A29

### 2-(1-Piperazinyl)-thiazol-4-carbonsäuremethylester-hydrochlorid

Zu der eisgekühlten Lösung von 8.0 g (15.752 mMol) 2-[4-(Phenylmethyl)-1-piperazinyl]-thiazol-4-carbonsäuremethylester in 60 ml 1,2-Dichlorethan gab man 4.0 ml (35.973 mMol) Chlorameisensäure-1-chlorethylester, rührte noch 20 Minuten bei 0 °C und kochte über Nacht unter Rückfluß, bevor man das Lösemittel abdestillierte. Der Rückstand wurde mit 60 ml Methanol versetzt und abermals 4 Stunden unter Rückfluß gekocht. Das Lösemittel wurde im Vakuum entfernt, der Rückstand mit 3 ml Methanol verrieben, dann abgenutscht. Nach dem Trocknen im Vakuumtrockenschrank erhielt man 2.5 g (60% der Theorie) an farblosen Kristallen vom R_{f} = 0.49 (FM D).

| | |
|---|---|
| ESI-MS: | (M+H)⁺ = 228; |
| | (M+Na)⁺ = 250 |

### Beispiel A30

### 2-[4-(Phenylmethyl)-1-piperazinyl]-thiazol-4-carbonsäure-hydrobromid

Zu der Lösung von 18.0 g (76.482 mMol) 1-(Aminothiocarbonyl)-4-(phenylmethyl)-piperazin in 300 ml Ethanol gab man 12.7 g (76.066 mMol) Brombrenztraubensäure und kochte 3 Stunden unter Rückfluß. Man ließ über Nacht stehen, nutschte das ausgefallene Festprodukt ab und wusch es mit Ethanol. Nach dem Trocknen erhielt man 23.0 g (79% der Theorie) an farblosen Kristallen vom R_{f} 0.10 (FM D).

| | |
|---|---|
| ESI-MS: | (M-H)⁻ = 302; |
| | (M+Na)⁺ = 326 |

### Beispiel A31

### 1-(Aminothiocarbonyl)-4-(phenylmethyl)-piperazin

Zu der eisgekühlten Lösung von 19.08 g (108.25 mMol) 1-(Phenylmethyl)-piperazin in 150 ml Dichlormethan tropfte man 12.596 g (108.247 mmol) *tert*-Butylisothiocyanat, wobei man die Reaktionstemperatur unter +5 °C hielt. Man rührte über Nacht bei Zimmertemperatur, befreite vom Lösemittel und kochte den verbliebenen Rückstand 1½ Stunden lang mit 100 ml konz. Salzsäure. Nach dem Erkalten neutralisierte man unter äußerer Kühlung mit 12M Natronlauge und extrahierte erschöpfend mit Dichlormethan. Die vereinigten Dichlormethanauszüge wurden über Natriumsulfat getrocknet und im Vakuum eingedampft. Man erhielt 25.2 g (99% der Theorie) an hellgelben Kristallen vom
R_{f} = 0.45 (FM D).

| | |
|---|---|
| ESI=MS: | (M+H)⁺ = 236; |
| | (M-H)⁻ = 234; |
| | (M+Na)⁺ = 258 |

### Beispiel A32

### 4-Methyl-1-(phenylmethyl)-2-piperazincarbonsäureethylester

Zu der Mischung aus 15.12 g (31.739 mmol) 1-(Phenylmethyl)-2-piperazincarbonsäureethylester-bis-(trifluoracetat), 20 ml DIEA und 250 ml THF tropfte man bei Zimmertemperatur die Lösung von 2.2 ml (35.029 mMol) lodmethan in 50 ml THF und rührte 4 weitere Stunden bei Zimmertemperatur. Man filtrierte, dampfte den Rückstand im Vakuum ein und chromatographierte den Rückstand an einer Kieselgelsäule unter Verwendung von FM II zum Eluieren. Nach der üblichen Aufarbeitung der geeigneten Fraktionen erhielt man 2.43 g (29% der Theorie) eines farblosen Öls, das ohne weitere Reinigung in der nächsten Stufen verwendet wurde.

Analog wurden die folgenden Verbindungen der allgemeinen Formel N-B-C erhalten:

| **N** | **B** | **C** | **Anmerkungen** | **% Ausb.** | **FM** | **R_{f}** | **MS** | **Fp.[°C]** |
|---|---|---|---|---|---|---|---|---|
| 4-(1,1-Dimethylethoxycarbohyl)-1-methyl-2-piperazincarbonsäure-ethylester | | | aus 4-(1,1-Dimethylethoxycarbonyl)-2-piperazincarbonsäure-ethylester, CH₃I und DIEA in THF | 79 | AcOEt | 0.58 | ESI: (M+H)⁺ = 273 | farbloses Öl |
| 4-(1,1-Dimethylethoxycarbonyl)-1-(phenylmethyl)-2-piperazincarbonsäure-ethylester | | | aus 4-(1,1-Dimethylethoxy-carbonyl)-2-piperazincarbonsäure-ethylester, PhCH₂Br und DIEA in THF | 90 | NN | 0.51 | ESI: (M+H)⁺ = 349 | |

### Beispiel A33

### 4-(1,1-Dimethylethoxycarbonyl)-2-piperazincarbonsäure-ethylester

Zu der Lösung von 17.07 g (0.108 Mol) 2-Piperazincarbonsäure-ethylester in 400 ml Ethanol tropfte man unter Eiskühlung 22.0 g (0.101 Mol) Pyrokohlensäure-di-*tert.-*butylester und rührte weitere 3 Stunden unter äußerer Kühlung mit Eis. Das Lösemittel wurde, zuletzt im Vakuum, abdestilliert und der verbleibende Rückstand zwischen Wasser und Essigsäureethylester verteilt. Die organische Phase wurde über Natriumsulfat getrocknet und im Vakuum eingeengt, der Rückstand unter Verwendung von Essigsäureethylester/Ethanol 95/5 v/v zum Eluieren säulenchromatographisch an Kieselgel gereinigt. Ausbeute: 11.798 g (42% der Theorie) einer farblosen Festsubstanz.

### Beispiel A34

### 1,4-Bis-(phenylmethyl)-2-piperazincarbonsäureethylester

Zu der 40 °C warmen Lösung von 52.190 g (217.141 mMol) *N*,*N*'-Dibenzylethylendiamin und 60 ml Triethylamin in 165 ml Toluol tropfte man unter kräftigem Rühren die Lösung von 56.441 g (217.141 mMol) 2,3-Dibrompropansäureethylester in 55 ml Toluol und rührte weitere 3 Stunden bei einer Badtemperatur von 80°C. Man ließ erkalten, filtrierte, wusch die Filtrate zweimal mit je 50 ml Wasser, dann einmal mit 100 ml gesättigter Kochsalzlösung, trocknete sie über Natriumsulfat und dampfte sie im Vakuum ein. Man erhielt 73.4 g (100% der Theorie) eines farblosen, viskosen Öls vom R_{f} 0.79 (FM MM), das ohne weitere Reinigung in der folgenden Stufe verwendet wurde.
ESI-MS: (M+H)⁺ = 339

### B. Herstellung der Endverbindungen

### (Referenz)Beispiel 1

### 4-{1-[3,5-Dibrom-N-[[4-(3,4-dihydro-2(1H)-oxochinazolin-3-yl)-1-piperidinyl]carbonyl]-D-tyrosyl]-4-piperidinyl}-1-piperazinessigsäureethylester

Die Mischung aus 954.048 mg (1.6 mmol) 3,5-Dibrom-*N*-[[4-(3,4-dihydro-2(1*H-*oxochinazolin-3-yl)-1-piperidinyl]carbonyl]-D-tyrosin, 955.898 mg (1.6 mmol) 4-(4-Piperidinyl)-1-piperazin-essigsäureethylester, 802.75 mg (2.5 mmol) TBTU, 216.208 mg (1.6 mmol) HOBt, 2.4 ml (14.02 mmol) DIEA und 8 ml THF-DMF-Gemisch (5/3 v/v) wurde über Nacht bei Raumtemperatur gerührt. Das Reaktionsgemisch wurde in 50 ml gesättigte wässerige Natriumhydrogencarbonat-Lösung eingerührt, der ausgefallene Feststoff an Kieselgel unter Verwendung von FM G zum Eluieren säulenchromatographisch gereinigt. Nach üblicher Aufarbeitung der geeigneten Eluate erhielt man 283 mg (21% der Theorie) eines farblosen, amorphen Produkts vom R_{f} 0.39 (FM G).

| | |
|---|---|
| IR (KBr): | 3405(NH, OH); 1731 (C=O) cm⁻¹ |
| ESI: | (M-H)⁻ = 830/832/834(Br₂); |
| | (M+Na)⁺ = 854/856/858(Br₂) |

Analog wurden die folgenden Verbindungen der allgemeinen Formel N-B-C hergestellt:

| **Lfd. Nr.** | **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|---|
| 1 | N2 | B2 | C5 | aus N2-CO-B2-OH, H-C5, TBTU, HOBt und DIEA in THF | 96 | G | 0.61 | ESI: (M+H)+ = 830/832/834; (M+HCO₂)⁻ = 874/876/878 (Br₂) | 3377 breit (NH, NH₂); 1734 (C=O) | farblose, amorphe Substanz |
| 3 | N2 | B2 | C11 | aus N2-CO-B2-OH, H-C11, TBTU, HOBt und DIEA in THF | 82 | G | 0.57 | ESI: (M+HCO₂)⁻ = 889/891/893 (Br₂) | 3446 breit (NH, NH₂); 1734 (C=O) | farblose, amorphe Substanz |
| 4 - | N2 | B2 | C26 | aus N2-CO-B2-OH, H-C26, TBTU, HOBt und DIEA in THF | 62 | D | 0.81 | ESI: (M-H)⁻ = 822/824/826 (Br₂); (M+Na)⁺ = 846/848/850 (Br₂) | | farblose Kristalle |
| 5 | N2 | B2 | C27 | aus N2-CO-B2-OH, H-C27 * 2 HBr, TBTU, HOBt und DIEA in THF | 65 | D | 0.79 | ESI: (M+Na)⁺ = 846/848/850 (Br₂) | | farblose Kristalle |
| 6 | N2 | B2 | C28 | aus N2-CO-B2-OH, H-C28, TBTU, HOBt und DIEA in THF | 38 | D | 0.81 | ESI: (M-H)⁻ = 807/809/811 (Br₂) | | farblose Kristalle |
| 7 | N2 | B2 | C30 | aus N2-CO-B2-OH, H-C30, TBTU, HOBt und DIEA in THF | 54 | D | 0.87 | ESI: (M+Na)⁺ = 873/875/877 (Br₂) | | farblose Kristalle |
| 8 | N2 | B2 | C31 | aus N2-CO-B2-OH, H-C31 * 2 HCl, TBTU, HOBt und DIEA in THF | 50 | D | 0.85 | ESI: (M+Na)⁺ = 900/902/904 (Br₂) | | farblose Kristalle |
| 9 | N2 | B2 | C32 | aus N2-CO-B2-OH, H-C32 * HCl, TBTU, HOBt und DIEA in THF | 52 | D | 0.88 | ESI: (M-H)⁻ = 807/809/811 (Br₂); (M+Na)⁺ = 831/833/835 (Br₂) | 1723 (C=O) | farblose Kristalle |
| 19 | N2 | B2 | C12 | aus N2-CO-B2-OH, H-C12, TBTU, HOBt und DIEA in THF | 65 | D | 0.49 | ESI: (M-H)⁻ = 871/873/875 (Br₂); (M+Na)⁺ = 895/897/899 (Br₂) | | farblose Kristalle |
| 20 | N2 | B2 | C1 | aus N2-CO-B2-OH, H-C1, TBTU, 843/845/847 HOBt und DIEA in THF | 58 | D | 0.72 | ESI: (M-H)⁻ = (Br₂); (M+H)⁺ = 845/847/849 (Br₂) | 1658 (C=O) | farblose Kristalle |

### 4-{1-[3,5-Dibrom-N-[[4-(3,4-dihydro-2(1H)-oxochinazolin-3-yl)-1-piperidinyl]carbonyl]-D-tyrosyl]-4-piperidinyl}-piperazin-essigsäure

Zu der Lösung von 85.0 mg (0.102 mmol) 4-{1-[3,5-Dibrom-*N*-[[4-(3,4-dihydro-2(1*H*)-oxochinazolin-3-yl)-1-piperidinyl]carbonyl]-D-tyrosyl]-4-piperidinyl}-1-piperazin-essigsäureethylester in 3.5 ml Methanol gab man bei Zimmertemperatur 0.5 ml 1M wässerige Natronlauge und rührte die Mischung 1 Stunde bei einer Reaktionstemperatur von 40°C. Man entfernte das Lösemittel im Vakuum und neutralisierte dann unter äußerer Kühlung mit Eis durch Zugabe von 0.5 ml 1 M Salzsäure. Man ließ 2 Stunden bei Zimmertemperatur stehen, bevor man die ausgefallenen Kristalle sammelte. Die Mutterlauge wurde abermals eingedampft, der Rückstand zur Entfernung anorganischer Salze mit wenigen Tropfen Wasser digeriert und nach zweistündigem Stehenlassen filtriert. Die vereinigten Festkörper wurden im Vakuum getrocknet, mit Diethylether verrieben und ergaben 80.0 mg (97% der Theorie) an farblosen Kristallen.

| | |
|---|---|
| ESI-MS: | (M+Na)⁺ = 826/828/830 (Br₂) |
| | (M-H)⁻ = 802/804/806 (Br₂) |

Entsprechend wurden die folgenden Verbindungen der allgemeinen Formel N-B-C erhalten:

| **Lfd. Nr.** | **N** | **B** | **C** | **Anmerkungen** | **% Ausb.** | **FM** | **R_{f}** | **MS** | **IR [cm⁻¹]** | **Fp. [°C]** |
|---|---|---|---|---|---|---|---|---|---|---|
| 2 | N2 | B2 | C6 | aus N2-CO-B2-C5 mit aq. 1M NaOH, dann aq. 1M HCl | 78 | G | 0.04 | ESI: (M-H)⁻ = 814/816/818 (Br₂); (M+H)⁺ = 816/818/820 (Br2); (M+HCO₂)⁻ = 859/861/863 (Br₂) | 3431 (NH, NH₂); 1653 (C=O) | farblose Kristalle |
| 10 | N2 | B2 | C33 | aus N2-CO-B2-C26 mit aq. 1M LiOH, dann aq. 1M HCl | 82 | I | 0.55 | ESI: (M-H)⁻ = 794/796/798 (Br₂); (M+Na)⁺ = 818/820/822 (Br₂) | | farblose Kristalle |
| 11 | N2 | B2 | C34 | aus N2-CO-B2-C27 mit aq. 1M LiOH, dann aq. 1M HCl | 76 | I | 0.54 | ESI: (M-H)⁻ = 794/796/798 (Br₂); (M+Na)⁺ = 818/820/822 (Br₂) | 1709, 1637 (C=O) | farblose Kristalle |
| 12 | N2 | B2 | C35 | aus N2-CO-B2-C28 mit aq. 1M LiOH, dann aq. 1M HCI | 76 | I | 0.54 | ESI: (M-H)⁻ = 793/795/797 (Br₂); (M+Na)⁺ = 817/819/821 (Br₂) | 1657 (C=O) | farblose Kristalle |
| 13 | N2 | B2 | C37 | aus N2-CO-B2-C30 mit aq. 1M LiOH, dann aq. 1M HCl | 86 | I | 0.56 | ESI: (M-H)⁻ = 821/823/825 (Br₂); (M+Na)⁺ = 845/847/849 (Br₂) | | farblose Kristalle |
| 14 | N2 | B2 | C38 | aus N2-CO-B2-C31 mit aq. 1M LiOH, dann aq. 1M HCl | 77 | I | 0.56 | ESI: (M-H)⁻ = 862/864/866 (Br₂); (M+Na)⁺ = 886/888/890 (Br₂) | | farblose Kristalle |
| 15 | N2 | B2 | C39 | aus N2-CO-B2-C32 mit aq. 1M LiOH, dann aq. (Br₂) 1M HCl | 71 | I | 0.57 | ESI: (M-H)⁻ = 793/795/797 | 1711 (C=O) | farblose Kristallen |
| 17 | N2 | B11 | C2 | aus N2-CO-B11-C1 mit aq. 0.1M LiOH, dann aq. 0.1M HCl | 83 | | | ESI: (M+H)⁺ = 696 | | farblose, amorphe Substanz |
| 18 | N2 | B2 | C4 | aus N2-CO-B2-C11 mit aq. 0.1M LiOH, dann aq. 0.1M HCl | 69 | M | 0.31 | ESI: (M-H)⁻ = 815/817/819 (Br₂); (M+Na)⁺ = 839/841/843 (Br₂) | | farblose Kristalle |
| 21 | N2 | B2 | C2 | aus N2-CO-B2-C1 mit aq. 1M LiOH, dann aq. 1M HCl | 73 | D | 0.05 | ESI: (M-H)⁻ = 815/817/819 (Br₂); (M+H)⁺ = 817/819/821 (Br₂); (M+Na)⁺ = 839/841/843 | | farblose Kristalle |

### Beispiel 3

### 4-{1-[3-(1-Naphthyl)-N-[[4-(2-oxo-1,3,4,5-tetrahydro-1,3-benzodiazepin-3-yl)-1-piperidinyl]carbonyl]-D-alanyl]-4-piperidinyl}-1-piperazinessigsäureethylester (Lfd. Nr. 16)

Eine Tetrahydrofuran-Lösung (20 ml) von 380.0 mg (0.84 mmol) 4-{1-[3-(1-Naphthyl)-D-alanyl]-4-piperidinyl}-1-piperazinessigsäureethylester wurde über einen Zeitraum von 40 Minuten zu einer auf -5 °C gekühlten und gerührten Suspension von 149.356 mg (0.91 mmol) CDT in 10 ml Tetrahydrofuran tropfenweise zugegeben. Die Reaktionsmischung wurde anschließend 1 Stunde bei -5 °C und 1 Stunde bei Raumtemperatur gerührt und mit der Suspension von 206.075 mg (0.84 mmol) 3-(4-Piperidinyl)-1,3,4,5-tetrahydro-1,3-benzodiazepin-2-on in 10 ml DMF versetzt. Um eine homogene Mischung zu erhalten, wurde das Tetrahydrofuran bei Normaldruck abdestilliert, nochmals 15 ml DMF zugegeben und das Gemisch anschließend 2 Stunden auf 100 °C erhitzt. Das Reaktionsgemisch wurde im Vakuum eingeengt, der Rückstand unter Verwendung einer im Hause entwickelten Gradientenmethode mittels Mischungen aus Dichlormethan, Methanol und konz. Ammoniak an Kieselgel säulenchromatographisch gereinigt, entsprechende Fraktionen mit Ether verrieben und der anfallende Feststoff (450.0 mg; 74% der Theorie) abgenutscht und getrocknet.
ESI-MS: (M+H)⁺ = 724

Die nachfolgenden Beispiele beschreiben die Herstellung pharmazeutischer Anwendungsformen. die als Wirkstoff eine beliebige Verbindung der allgemeinen Formel (I) enthalten:

### Beispiel I

### Kapseln zur Pulverinhalation mit 1 mg Wirkstoff

**Zusammensetzung:**

| 1 Kapsel zur Pulverinhalation enthält: | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

### Inhalationslösung für Respimat^{®} mit 1 mg Wirkstoff

**Zusammensetzung:**

| 1 Hub enthält: | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

### Inhalationslösung für Vernebler mit 1 mg Wirkstoff

**Zusammensetzung:**

| 1 Fläschchen enthält: | |
|---|---|
| Wirkstoff | 0.1 g |
| Natriumchlorid - | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

### Treibgas-Dosieraerosol mit 1 mg Wirkstoff

**Zusammensetzung:**

| 1 Hub enthält: | |
|---|---|
| Wirkstoff | 1.0 mg |
| Lecithin | 0.1% |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

### Nasalspray mit 1 mg Wirkstoff

**Zusammensetzung:**

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellunqsverfahren:

Der Wirkstoff und die Hilfsstoffe werden in Wasser gelöst und in ein entsprechendes Behältnis abgefüllt.

### Beispiel VI

### Injektionslösung mit 5 mg Wirksubstanz pro 5 ml

**Zusammensetzung:**

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

### Injektionslösung mit 100 mg Wirksubstanz pro 20 ml

**Zusammensetzung:**

| | |
|---|---|
| Wirksubstanz | 100 mg |
| Monokaliumdihydrogenphosphat = KH₂PO₄ | 12 mg |
| Dinatriumhydrogenphosphat = Na₂HPO₄·2H₂O | 2 mg |
| Natriumchlorid | 180 mg |
| Human-Serum-Albumin | 50 mg |
| Polysorbat 80 | 20 mg |
| Wasser für Injektionszwecke ad | 20 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

### Lyophilisat mit 10 mg Wirksubstanz

**Zusammensetzung:**

| | |
|---|---|
| Wirksubstanz | 10 mg |
| Mannit | 300 mg |
| Human-Serum-Albumin | 20 mg |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen;

in Vials abfüllen; gefriertrocknen.

**Lösungsmittel für Lyophilisat:**

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

### Tabletten mit 20 mg Wirksubstanz

**Zusammensetzung:**

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

### Beispiel X

### Kapseln mit 20 mg Wirksubstanz

**Zusammensetzung:**

| | |
|---|---|
| Wirksubstanz | 20 mg |
| Maisstärke | 80 mg |
| Kieselsäure. hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Grösse 3 abfüllen.

### Beispiel XI

### Zäpfchen mit 50 mg Wirksubstanz

**Zusammensetzung:**

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgiessen.

### Beispiel XII

### Injektionslösung mit 10 mg Wirksubstanz pro 1 ml

**Zusammensetzung:**

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Carbonsäuren und Ester der allgemeinen Formel 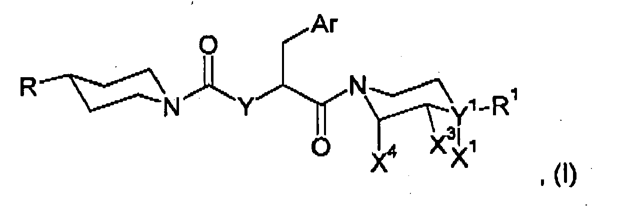 in der
R die 2-Oxo-1,3,4,5-tetrahydro-1,3-benzodiazepin-3-yl-Gruppe,
Ar die 3,5-Dibrom-4-hydroxyphenyl- 4-Amino-3,5-dibromphenyl-, 4-Brom-3,5-dimethylphenyl-, 3,5-Dichlor-4-methylphenyl-, 3,4-Dibromphenyl-, 3-Brom-4,5-dimethylphenyl-, 3,5-Dibrom-4-methylphenyl-, 3-Chlor-4-methylphenyl-, 3,4-Difluorphenyl-, 4-Hydroxyphenyl-, 1-Naphthyl-, 3,5-Dibrom-4-fluorphenyl-, 3,5-Bis-(trifluormethyl)-phenyl-, 3,4,5-Trimethylphenyl-, 3-(Trifluormethyl)-phenyl-, 3,5-Dimethyl-4-methoxyphenyl-, 4-Amino-3,5-dichlorphenyl-, 2,4-Bis-(trifluormethyl)phenyl-, 3,4,5-Tribromphenyl-, 3,4-Dimethoxyphenyl-, 3,4-Dichlorphenyl-, 4-Brom-3,5-dichlorphenyl-, 2-Naphthyl-, 2,3-Dihydrobenzo[b]fur-5-yl-, 1,2,3,4-Tetrahydro-1-naphthyl- oder 2,3-Dichlorphenylgruppe,
Y die Methylen- oder die -NH-Gruppe,
Y¹ das Kohlenstoff- oder das Stickstoffatom,
X¹ ein freies Elektronenpaar, wenn Y¹ das Stickstoffatom bedeutet, oder, sofern Y¹ das Kohlenstoffatom ist, das Wasserstoffatom, die Carbonsäure- oder die Methoxycarbonylgruppe und
R¹ einen Rest der allgemeinen Formel 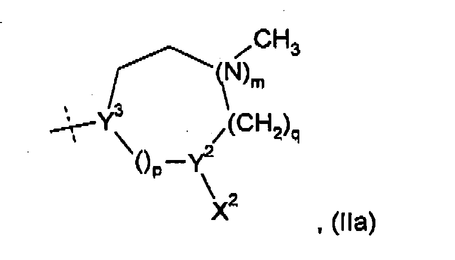 in der
Y² das Kohlenstoffatom oder, sofern m den Wert 0 annimmt, auch das Stickstoffatom,
Y³, das von Y¹ stets verschieden ist, das Kohlenstoff- oder das Stickstoffatom,
X² eine Gruppe der allgemeinen Formel
CH₂CO₂R² , (III)
in der
R² das Wasserstoffatom oder einen geradkettigen oder verzweigten C₁₋₄-Alkylrest darstellt,
oder, sofern Y² das Kohlenstoffatom ist, auch das Wasserstoffatom oder die gegebenenfalls mit Methanol oder Ethanol veresterte Carbonsäuregruppe,
m die Zahlen 0 oder 1,
p und q jeweils die Zahlen 0, 1 oder 2,
wobei die Summe von m, p und q die Werte 1 oder 2 annehmen kann,
bedeuten,
oder einen der Reste 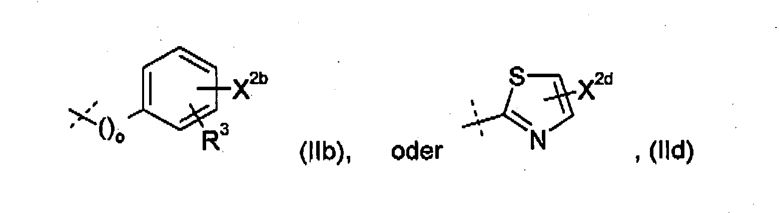 worin
X^{2b} das Wasserstoffatom oder die gegebenenfalls mit Methanol oder Ethanol veresterte Carbonsäuregruppe,
X^{2d} das Wasserstoffatom oder die gegebenenfalls mit Methanol veresterte Carbonsäuregruppe,
o die Zahlen 0, 1 oder 2 und
R³ das Wasserstoffatom oder die Trifluormethylgruppe darstellen,
wobei die vorstehend genannten Alkylgruppen oder die in den vorstehend genannten Resten enthaltenen Alkylgruppen, sofern nichts anderes angegeben wurde, 1 bis 4 Kohlenstoffatome enthalten und geradkettig oder verzweigt sein können,
deren Tautomere, deren Diastereomere, deren Enantiomere und deren Salze.

2. Folgende Carbonsäuren und Ester der allgemeinen Formel I gemäß Anspruch 1:
(1) 1'-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-[1,4']bipiperidinyl-4-essigsäuremethylester,
(2) 1'-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-[1,4']bipiperidinyl-4-essigsäure,
(3) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-1-piperidinessigsäureethylester,
(4) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-benzoesäureethylester,
(5) 3-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-benzoesäureethylester,
(6) 4-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-benzoesäuremethylester,
(7) 4-{2-[1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl]-ethyl}benzoesäuremethylester,
(8) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-3-(trifluormethyl)-benzoesäuremethylester,
(9) 3-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-benzoesäuremethylester,
(10) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-benzoesäure,
(11) 3-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-benzoesäure,
(12) 4-{1-[4-Amino-3,5-dibrom-*N*-[[4-(2-oxo-1,3,4,5-terahydro-1,3-benzodiazepin-3-yl)-1-piperidinyl]carbonyl]-D-phenylalanyl]-4-piperidinyl}-benzoesäure,
(13) 4-{2-[1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl]-ethyl}benzoesäure,
(14) 4-{4-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-1-piperazinyl}-3-(trifluormethyl)-benzoesäure,
(15) 3{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-benzoesäure,
(16) 4-{1-[3-(1-Naphthyl)-*N*-[[4-(2-oxo-1,3,4,5-tetrahydro-1,3-benzodiazepin-3-yl)-1-piperidinyl]carbonyl]-D-alanyl]-4-piperidinyl}-1-piperazinessigsäureethylester,
(17) 4-{1-[3-(1-Naphthyl)-*N*-[[4-(2-oxo-1,3,4,5-tetrahydro-1,3-benzodiazepin-3-yl)-1-piperidinyl]carbonyl]-D-alanyl]-4-piperidinyl}-1-piperazinessigsäure,
(18) 4-{4-[4-Amino-3,5-dibrom-*N*-[[4-(2-oxo-1,3,4,5-tetahydro-1,3-benzodiazepin-3-yl)-1-piperidinyl]carbonyl]-D-phenylalanyl]-1-piperazinyl}-1-piperidinessigsäure,
(19) 4-{1-[4-Amino-3,5-dibrom-*N*-[[4-(2-oxo-1,3,4,5-tetrahydro-1,3-benzodiazepin-3-yl)-1-piperidinyl]-D-phenylalanyl]-4-piperidinyl}-1-piperazinessigsäure-1,1-d imethylethylester,
(20) 4-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-1-piperazinessigsäureethylester,
(21) 4-{1-[4-Amino-3,5-dibrom-*N*-[[4-(2-oxo-1,3,4,5-tetrahydro-1,3-benzodiazepin-3-yl)-1-piperidinyl]carbonyl]-D-phenylalanyl]-4-piperidinyl}-1-piperazinessigsäure,
und deren Salze.

3. Physiologisch verträgliche Salze der Verbindungen nach einem der Ansprüche 1 bis 2 mit anorganischen oder organischen Säuren oder Basen.

4. Arzneimittel, enthaltend eine Verbindung nach einem der Ansprüche 1 bis 2 oder ein physiologisch verträgliches Salz gemäß Anspruch 3 neben gegebenenfalls einem oder mehreren inerten Trägerstoffen und/oder Verdünnungsmitteln.

5. Verwendung einer Verbindung nach einem der Ansprüche 1 bis 3 zur Herstellung eines Arzneimittels zur akuten oder prophylaktischen Behandlung von Kopfschmerzen, zur Behandlung des nicht-insulinabhängigen Diabetes mellitus, von cardiovaskulären Erkrankungen, der Morphintoleranz, von Erkrankungen der Haut, entzündlichen Erkrankungen, allergischer Rhinitis, Asthma, von Erkrankungen, die mit einer überschießenden Gefäßerweiterung und dadurch bedingter verringerter Gewebedurchblutung einhergehen, der akuten oder präventiven Behandlung von menopausalen Hitzewallungen östrogendefizienter Frauen oder zur Behandlung von Schmerzzuständen.

## Claims

1. Carboxylic acids and esters of general formula 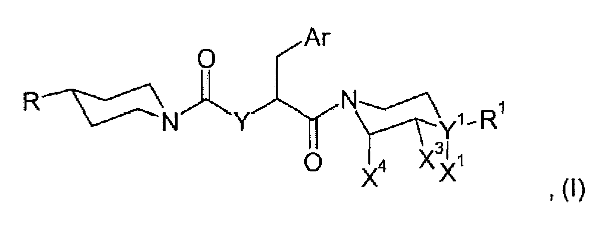 wherein
R denotes the 2-oxo-1,3,4,5-tetrahydro-1,3-benzodiazepin-3-yl group,
Ar denotes the 3,5-dibromo-4-hydroxyphenyl, 4-amino-3,5-dibromophenyl, 4-bromo-3,5-dimethylphenyl, 3,5-dichloro-4-methylphenyl, 3,4-dibromophenyl, 3-bromo-4,5-dimethylphenyl, 3,5-dibromo-4-methylphenyl, 3-chloro-4-methylphenyl, 3,4-difluorophenyl, 4-hydroxyphenyl, 1-naphthyl, 3,5-dibromo-4-fluorophenyl, 3,5-bis-(trifluoromethyl)-phenyl, 3,4,5-trimethylphenyl, 3-(trifluoromethyl)-phenyl, 3,5-dimethyl-4-methoxyphenyl, 4-amino-3,5-dichlorophenyl, 2,4-bis-(trifluoromethyl)-phenyl, 3,4,5-tribromophenyl, 3,4-dimethoxyphenyl, 3,4-dichlorophenyl, 4-bromo-3,5-dichlorophenyl, 2-naphthyl, 2,3-dihydrobenzo[b]fur-5-yl, 1,2,3,4-tetrahydro-1-naphthyl or 2,3-dichlorophenyl group,
Y denotes the methylene or the -NH- group,
Y¹ denotes the carbon or the nitrogen atom,
X¹ denotes a free electron pair, if Y¹ denotes the nitrogen atom, or, if Y¹ is the carbon atom, the hydrogen atom, the carboxylic acid or the methoxycarbonyl group and
R¹ denotes a group of general formula 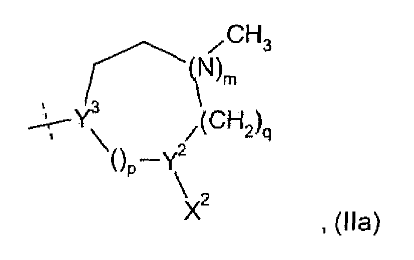 wherein
Y² denotes the carbon atom or, if m assumes the value 0, also the nitrogen atom,
Y³, which is always different from Y¹, denotes the carbon or the nitrogen atom,
X² denotes a group of general formula
CH₂CO₂R² , (III)
wherein
R² denotes the hydrogen atom or a straight-chain or branched C₁₋₄-alkyl group,
or, if Y² is the carbon atom, also denotes the hydrogen atom or the carboxylic acid group optionally esterified with methanol or ethanol,
m denotes the numbers 0 or 1,
p and q in each case denote the numbers 0, 1 or 2,
while the sum of m, p and q may assume the values 1 or 2,
or one of the groups 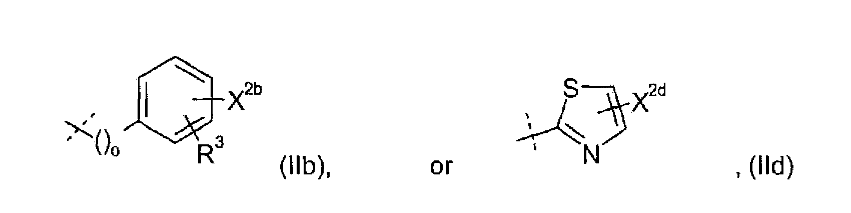 wherein
X^{2b} denotes the hydrogen atom or the carboxylic acid group optionally esterified with methanol or ethanol,
X^{2d} denotes the hydrogen atom or the carboxylic acid group optionally esterified with methanol,
o denotes the numbers 0, 1 or 2 and
R³ denotes the hydrogen atom or the trifluoromethyl group,
while, unless otherwise stated, the above-mentioned alkyl groups or the alkyl groups contained in the above-mentioned groups contain 1 to 4 carbon atoms and may be straight-chain or branched,
the tautomers, the diastereomers, the enantiomers and the salts thereof.

2. The following carboxylic acids and esters of general formula I according to claim 1:
(1) methyl 1'-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-[1,4']bipiperidinyl-4-acetate,
(2) 1'-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-[1,4']bipiperidinyl-4-acetic acid,
(3) ethyl 4-{4-[4-amino-3,5-dibromo-*N*-[[4-(2-oxo-1,3,4,5-tetrahydro-1,3-benzodiazepin-3-yl)-1-piperidinyl]carbonyl]-D-phenylatanyl]-1-piperazinyl}-1-piperidineacetate,
(4) ethyl 4-{4-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-1-piperazinyl}-benzoacetate,
(5) ethyl 3-{4-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-1-piperazinyl}-benzoate,
(6) methyl 4-{1-[4-amino-3,5-dibromo-*N*-[[4-(2-oxo-1,3,4,5-tetrahydro-1,3-benzodiazepin-3-yl)-1-piperidinyl]carbonyl]-D-phenylainyl]-4-piperidinyl}-benzoate,
(7) methyl 4-{2-[1-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-4-piperidinyl]-ethyl}-benzoate,
(8) methyl 4-{4-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-1-piperazinyl}-3-(trifluoromethyl)-benzoate,
(9) methyl 3-{1-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-4-piperid inyl}-benzoate,
(10) 4-{4-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-1-piperazinyl}-benzoic acid,
(11) 3-{4-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-1-piperazinyl}-benzoic acid,
(12) 4-{1-[4-amino-3,5-dibromo-*N*-[[4-(2-oxo-1,3,4,5-terahydro-1,3-benzodiazepin-3-yl)-1-piperidinyl]carbonyl]-D-phenylalanyl]-4-piperidinyl}-benzoic acid,
(13) 4-{2-[1-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-4-piperidinyl]-ethyl}-benzoic acid,
(14) 4-{4-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-1-piperazinyl}-3-(trifluoromethyl)-benzoic acid,
(15) 3-{1-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-4-piperidinyl}-benzoic acid,
(16) ethyl 4-{1-[3-(1-naphthyl)-*N*-[[4-(2-oxo-1,3,4,5-tetrahydro-1,3-benzodiazepin-3-yl)-1-piperidinyl]carbonyl]-D-alanyl]-4-piperid inyl}-1-piperazineacetate,
(17) 4-{1-[3-(1-naphthyl)-*N*-[[4-(2-oxo-1,3,4,5-tetrahydro-1,3-benzodiazepin-3-yl)-1-piperidinyl]carbonyl]-D-alanyl]-4-piperidinyl}-1-piperazineacetic acid,
(18) 4-{4-[4-amino-3,5-dibromo-*N*-[[4-(2-oxo-1,3,4,5-tetrahydro-1,3-benzodiazepin-3-yl)-1-piperidinyl]carbonyl]-D-phenylalenyl]-1-piperazinyl}-1-piperidineacetic acid,
(19) 1,1-dimethylethyl 4-{1-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-4-piperidinyl}-1-piperazineacetate,
(20) ethyl 4-{1-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-4-piperidinyl}-1-piperazineacetate,
(21) 4-{1-[4-amino-3,5-dibromo-*N*-[[4-(2-oxo-1,3,4,5-tetrahydro-1,3-benzodiazepin-3-yl)-1-piperidinyl]carbonyl]-D-phenylalanyl]-4-piperidinyl}-1-piperazineacetic acid,
and the salts thereof.

3. Physiologically acceptable salts of the compounds according to one of claims 1 to 2 with inorganic or organic acids or bases.

4. Medicaments containing a compound according to one of claims 1 to 2 or a physiologically acceptable salt according to claim 3 optionally together with one or more inert carriers and/or diluents.

5. Use of a compound according to one of claims 1 to 3 for preparing a medicament for the acute or prophylactic treatment of headaches, for treating non-insulin-dependent diabetes mellitus, cardiovascular diseases, morphine tolerance, skin diseases, inflammatory diseases, allergic rhinitis, asthma, diseases accompanied by excessive vasodilatation and resultant reduced circulation of the blood, for acute or preventive treatment of the menopausal hot flushes in oestrogen-deficient women or for treating pain.

## Revendications

1. Acides carboxyliques et esters de formule générale 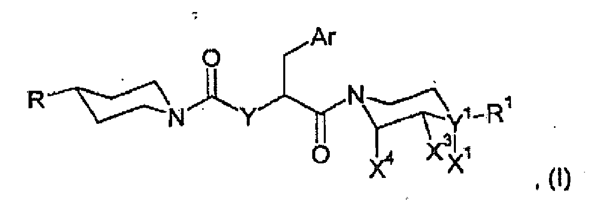 où
R représente le groupe 2-oxo-1,3,4,5-tétrahydro-1,3-benzodiazépin-3-yle, Ar représente le groupe 3,5-dibromo-4-hydroxyphényle, 4-amino-3,5-dibromophényle, 4-bromo-3,5-diméthylphényle, 3,5-dichloro-4-méthylphényle, 3,4-dibromophényle, 3-bromo-4,5-diméthylphényle, 3,5-dibromo-4-méthylphényle, 3-chloro-4-méthylphényle, 3,4-difluorophényle, 4-hydroxyphényle, 1-naphtyle, 3,5-dibromo-4-fluorophényle, 3,5-bis-(trifluorométhyl)-phényle, 3,4,5-triméthylphényle, 3-(trifluorométhyl)-phényle, 3,5-diméthyl-4-méthoxyphényle, 4-amino-3,5-dichlorophényle, 2,4-bis-(trifluorométhyl)-phényle, 3,4,5-tribromophényle, 3,4-diméthoxyphényle, 3,4-dichlorophényle, 4-bromo-3,5-dichlorophényle, 2-naphtyle, 2,3-dihydrobenzo[b]fur-5-yle, 1,2,3,4-tétrahydro-1-naphtyle
ou 2,3-dichlorophényle,
Y représente le groupe méthylène ou -NH-,
Y¹ représente l'atome de carbone ou d'azote,
X¹ représente un paire d'électrons libres quand Y¹ représente l'atome d'azote, ou, dans la mesure où Y¹ est l'atome de carbone, l'atome d'hydrogène, le groupe acide carboxylique ou méthoxycarbonyle, et
R¹ représente un groupement de formule générale 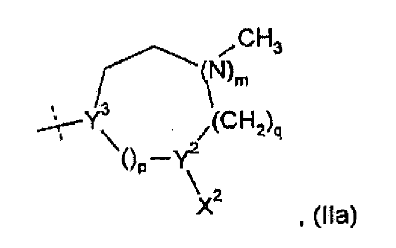 où
Y² représente l'atome de carbone ou, dans la mesure où m prend la valeur 0, également l'atome d'azote,
Y³, qui est toujours différent de Y¹, représente l'atome de carbone ou d'azote,
X² représente un groupe de formule générale
CH₂CO₂R² (III)
où
R² représente l'atome d'hydrogène ou un groupement C₁₋₄-alkyle linéaire ou ramifié,
ou, dans la mesure où Y² est l'atome de carbone, également l'atome d'hydrogène
ou le groupe acide carboxylique éventuellement estérifié avec le méthanol ou l'éthanol,
m représente les nombres 0 ou 1,
p et q représentent chacun les nombres 0, 1 ou 2,
où la somme de m, p et q peut prendre les valeurs 1 ou 2,
ou l'un des groupements 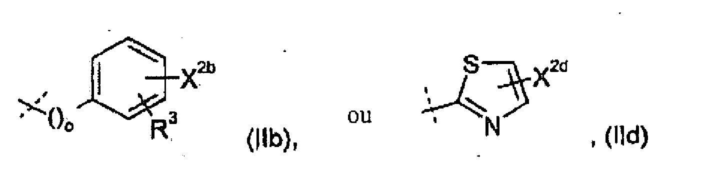 où
X^{2b} représente l'atome d'hydrogène ou le groupe acide carboxylique éventuellement estérifié avec le méthanol ou l'éthanol,
X^{2d} représente l'atome d'hydrogène ou le groupe acide carboxylique éventuellement estérifié avec le méthanol,
o représente les nombres 0, 1 ou 2 et
R³ représente l'atome d'hydrogène ou le groupe trifluorométhyle,
où les groupes alkyle cités précédemment ou les groupes alkyle contenus dans les groupements cités précédemment, sauf indication contraire, contiennent 1 à 4 atomes de carbone et peuvent être linéaires ou ramifiés,
leurs tautomères, leurs diastéréoisomères, leurs énantiomères et leurs sels.

2. Acides carboxyliques et esters de formule générale 1 selon la revendication 1 suivants :
(1) 1'-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-[1,4']bipipéridinyl-4-acétate de méthyle,
(2) acide 1'-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-[1,4']bipipéridinyl-4-acétique,
(3) 4-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazjnyl}-1-piperidine-acétate d'éthyle,
(4) 4-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazinyl}-benzoate d'éthyle,
(5) 3-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazinyl}-benzoate d'éthyle,
(6) 4-{1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl}-benzoate de méthyle,
(7) 4-{2-[1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl]-éthyl}benzoate de méthyle,
(8) 4-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazinyl}-3-(trifluorométhyl)benzoate de méthyle,
(9) 3-{1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl}-benzoate de méthyle,
(10) acide 4-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazinyl}benzoïque,
(11) acide 3-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazinyl}benzoïque,
(12) acide 4-{1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl}benzoïque,
(13) acide 4-{2-[1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl]-éthyl}-benzoïque,
(14) acide 4-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazinyl}-3-(trifluorométhyl)benzoïque,
(15) acide 3-{1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl}benzoïque,
(16) 4-{1-[3-(1-naphtyl)-*N*-[[4-(2-oxo-1,3,4,5-tétrahydro-1,3-benzodiazépin-3-yl)1-pipéridinyl]carbonyl]-D-alanyl]-4-pipéridinyl}-1-pipérazine-acétate d'éthyle,
(17) acide 4-{1-[3-(1-naphtyl)-N-[[4-(2-oxo-1,3,4,5-tétrahydro-1,3-benzodiazépin-3-yl)-1-pipéridinyl]carbonyl]-D-alanyl]-4-pipéridinyl}-1-pi pérazi ne-acétique,
(18) acide 4-{4-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-1-pipérazinyl}-1-pipéridine-acétique
(19) 4-{1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl}-1-pipérazine-acétate de 1,1-diméthyléthyle,
(20) 4-{1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl}-1-pipérazine-acétate d'éthyle,
(21) acide 4- {1-[4-amino-3,5-dibromo-*N*-[[4-(2-oxo-1,3,4,5-tétrahydro-1,3-benzodiazépin-3-yl)-1-pipéridinyl]carbonyl]-D-phénylalanyl]-4-pipéridinyl}-1-pipérazine-acétique,
et leurs sels.

3. Sels physiologiquement acceptables des composés selon l'une des revendications 1 à 2 avec des acides ou bases inorganiques ou organiques.

4. Médicament contenant un composé selon l'une des revendications 1 à 2 ou un sel physiologiquement acceptable selon la revendication 3 outre éventuellement un ou plusieurs vecteurs et/ou diluants inertes.

5. Utilisation d'un composé selon l'une des revendications 1 à 3 pour la production d'un médicament pour le traitement aigu ou prophylactique des maux de tête, pour le traitement du diabète sucré non insulino-dépendant, des maladies cardiovasculaires, de la tolérance à la morphine, des maladies de la peau, des maladies inflammatoires, de la rhinite allergique, de l'asthme, des maladies qui s'accompagnent d'un élargissement excessif des vaisseaux et d'une irrigation sanguine des tissus réduite qui en résulte, le traitement aigu ou préventif des bouffées de chaleur ménopausiques des femmes ayant une carence en estrogènes ou pour le traitement des états douloureux.
